# EUROPEAN PATENT APPLICATION

(11) **EP 3 653 714 A1**
(43) Date of publication of application: **20.05.2020**
(21) Application number: 19203386.8
(22) Date of filing: 21.11.2014
(51) Int. Cl.: C12N 15/63, A61K 35/761, A61K 45/00, A61P 35/00

(54) **ADENOVIRUS EXPRESSING IMMUNE CELL STIMULATORY RECEPTOR AGONIST(S)**

(30) Priority: 22.11.2013 US 201361907860 P
(62) Divisional of application: 14864921.3
(71) Applicant: DNAtrix, Inc., Houston, TX 77021 (US); Board Of Regents, The University Of Texas System, Austin, TX 78701 (US)
(72) Inventor: TUFARO, Frank, Rancho Santa Fe, CA California 92067 (US); FUEYO-MARGARETO, Juan, Houston, TX Texas 77081 (US); GOMEZ-MANZANO, Candelaria, Houston, TX Texas 77081 (US); CONRAD, Charles, deceased (US); YUNG, W.K., Alfred, Houston, TX Texas 77005 (US); JIANG, Hong, Houston, TX Texas 77025 (US)
(74) Representative: Potter Clarkson

(57) **Abstract**

A replication competent oncolytic virus comprising a heterologous nucleic acid inserted into a nonessential region of the adenovirus genome, said nucleic acid comprising a sequence encoding an OX40 (CD134) agonist operatively linked to a transcriptional control element.

## Description

### BACKGROUND

### I. Field of Invention

The present invention relates generally to the fields of oncology and cancer therapy. More particularly, it concerns replicative oncolytic viruses genetically modified to express an immune cell stimulatory receptor agonist such as OX40 ligand (OX40L).

### II. Description of Related Art

Cancer remains one of the leading causes of morbidity and mortality in humans worldwide. Although surgery, chemotherapy and radiation have been utilized with some success to cure cancer, novel strategies are needed. Viruses that replicate in tumor cells better than in normal cells have shown promise as oncolytic agents. The feasibility of gene transfer and tumor lysis using adenoviruses has been well established.

There remains a need for additional anti-cancer therapeutics.

### SUMMARY

The present invention relates to novel replication-competent oncolytic viruses expressing one or more immune cell stimulatory receptor agonists, pharmaceutical compositions comprising the replication-competent oncolytic adenovirus and their use in treating a variety of cancers. In preferred embodiments, the replication-competent oncolytic virus is an adenovirus. The replication-competent oncolytic virus will present the immune cell stimulatory receptor agonist from the first replication cycle, triggering a persistent effector anti-tumor immune response by activating lymphocytes that recognize tumor antigens and reversing the immune suppressive environment surrounding the tumor. In certain aspects, administration of the replication-competent oncolytic virus such as adenovirus to a subject with cancer provides an enhanced and even synergistic anti-tumor immunity compared to the unmodified virus (i.e. not expressing an immune cell stimulatory receptor agonist) and the immune cell stimulatory receptor agonist when administered separately. In related aspects, the anti-tumor effects of the replication-competent oncolytic virus persist even after clearance of the virus and even extend to one or more non-infected tumors.

In certain aspects, the replication-competent oncolytic virus expresses an immune cell stimulatory receptor agonist from a heterologous nucleic acid incorporated into a non-essential region of the viral genome, the heterologous nucleic acid comprising a nucleic acid sequence encoding the immune cell stimulatory receptor agonist. In some embodiments, the replication-competent oncolytic virus is an adenovirus and expression of the immune cell stimulatory receptor agonist is under the control of an endogenous adenovirus promoter such as the E3 promoter or a late adenoviral promoter such as the major late promoter. In other embodiments, the replication-competent oncolytic virus is an adenovirus and the nucleic acid encoding the immune cell stimulatory receptor agonist is under the control of (i.e. operatively linked to) a non-adenoviral transcriptional and/or translational control sequence such as an enhancer, promoter and/or leader sequence from cytomegalovirus (CMV) (e.g. a CMV promoter), rous sarcoma virus (RSV) (e.g. an RSV promoter) or simian virus 40 (SV40) (e.g. an SV40 promoter). A "heterologous" region of the construct is an identifiable segment of nucleic acid within a larger nucleic acid molecule that is not found in association with the larger molecule in nature.

In several embodiments, the replication-competent oncolytic virus expresses an agonist of an immune cell stimulatory receptor selected from the group consisting of: CD28, OX40 (CD134), glucocorticoid-induced TNF-receptor (GITR), CD137 (4-1BB), and herpes virus entry mediator A (HVEM). OX40, GITR, CD137 and HVEM are members of the tumor necrosis factor receptor (TNFR) family that are inducibly expressed upon T cell activation and accordingly induce costimulation on activated effector T cells and memory T cells. Stimulation through CD28 must be induced by professional antigen presenting cells (APCs) such as dendritic cells and macrophages; costimulation through TNFR family members such as OX40 and CD137 can be induced by expression of their respective ligands on nonhematopoietic cells in the periphery. In a preferred embodiment, the replication-competent oncolytic virus is an adenovirus.

CD28 is the most prominent costimulation receptor and is constitutively expressed on T cells and plays a critical role in stimulating naive T cells for proliferation, effector function and differentiation. In one embodiment, the replication-competent oncolytic virus (e.g. adenovirus) expresses an agonist of a CD28 agonist such as human CD80 (B7.1), GenBank Accession Nos. NM_005191 (mRNA) and NP_005182 (protein) or CD86 (B7.2), GenBank Accession No. NM_175862 (mRNA) and accession no. P42081 in the Swiss-Prot database.

GITR is expressed constitutively at high levels on regulatory T cells and activated CD4+ and CD8+ T cells. Engagement of GITR by its receptor GITR ligand (GITRL) has been shown to dampen the suppressive effects of regulatory T cells and co-activate effector T cells. In one embodiment, the replication-competent oncolytic virus (e.g. adenovirus) expresses an agonist of GITR such as human GITRL, NCBI database Entrez Gene ID: 8995.

4-1BB (CD37) is expressed on the surface of activated CD4+ and CD8+ T cells, on natural killer cells, monocytes and resting dendritic cells. Engagement of 4-1BB with its ligand, 4-1BB ligand (4-1BBL) plays a role in T cell survival and the establishment of long-term immunological memory and selectively promotes type 1 cytokines such as IL-2, IFN-γ and TNF-α. In one embodiment, the replication-competent oncolytic virus (e.g. adenovirus) expresses an agonist of 4-1BB such as human 4-1BBL, the full amino acid sequence of which can be found under accession no. P41273 in the Swiss-Prot database.

HVEM is expressed in peripheral blood T cells, B cells and monoctyes. Engagement of HVEM with its receptor LIGHT costimulates T- and B-cell activation, upregulates apoptotic genes and induces cytokine production, particularly, of IFN-γ and TNFα. In one embodiment, the replication-competent oncolytic virus (e.g. adenovirus) expresses an agonist of HVEM such as human lymphotoxin-like (LIGHT), the full amino acid sequence of which can be found under accession no. 043557 in the Swiss-Prot database.

In a preferred embodiment, the replication-competent oncolytic virus comprises a heterologous nucleic acid encoding an OX40 agonist. An OX40 agonist interacts with the XO40 receptor on e.g. activated T cells during or shortly after priming by a tumor or adenoviral antigen and results in an enhanced and prolonged immune response to the tumor. Preferably, the OX-40 agonist is expressed on the surface of the host cell (e.g. tumor cell) following infection of the cell with the replication competent oncolytic virus. In one preferred embodiment, the replication-competent oncolytic virus is an adenovirus comprising a heterologous nucleic acid encoding an OX40 agonist.

In a particularly preferred embodiment, the replication-competent oncolytic virus comprises a heterologous nucleic acid encoding OX40 ligand (OX40L or gp34) or an OX40 receptor-binding fragment of OX40L or an OX40L fusion protein such as those described in US Patent No. 7,959,925, the content of which is incorporated herein by reference. In one particularly preferred embodiment, the replication-competent oncolytic virus is an adenovirus comprising a heterologous nucleic acid encoding OX40L. OX40L, also known as gp34, like other TNF superfamily members, exists as a homotrimer on the surface of activated B cells, T cells, dendritic cells and endothelial cells. Binding of OX40L to OX40 (CD134) sustains the initial CD28-mediated T cell response and promotes both T-cell differentiation and survival. In particular, engagement of OX40 by its natural ligand OX40L or other OX40 agonists has been shown to provide key signals that can augment CD4 and CD8 T-cell responses. OX40 signaling also controls regulatory T cell differentiation and suppressive function. Importantly, numerous studies have highlighted the ability of OX40-specific agonists to enhance antitumor immunity or ameliorate autoimmune disease, respectively. On the basis of these studies, the development of OX40- and OX40L-specific reagents has been pursued for clinical use. Studies over the past decade have demonstrated that OX40 agonists enhance anti-tumor immunity in preclinical models using immunogenic tumors; however, treatment of poorly immunogenic tumors has been less successful. Combining strategies that prime tumor-specific T cells together with OX40 signaling could generate and maintain a therapeutic anti-tumor immune response. The amino acid sequence of human OX40L is described at GenBank Accession Number NP_003317.1. Full cDNA encoding human OX40L is at NCBI Reference Sequence: NM_003326.3. Additional OX40L sequences are further disclosed in e.g. SwissProt Accession Number P23510. Human OX40L shares 46% amino acid sequence identity with its mouse counterpart.

Other OX40 agonists that can be expressed by the replication-competent oncolytic adenovirus include antibodies against OX40 such as those described in US Patent Nos. 6,312,700, 7,504,101, 7,291,331, and 7,807,156, the entire contents of each of which are incorporated herein by reference. Specific non-limiting examples of OX40 antibody include 112F32, 112V8, 112Y55, 112Y131, 112Z5, mAb 315, mAb131, mAb 2G2, IF7, ACT35, mAb L106 and mAb OX86. Other OX40 agonists include those described in U.S. Patent Application Publication No. US20060281072, the entire content of which is incorporated herein by reference.

DNA encoding an immune cell stimulatory receptor agonist can be inserted e.g. at any nonessential location in the oncolytic virus so long as the oncolytic virus remains replication competent. In one embodiment, the oncolytic virus is an adenovirus with a heterologous nucleic acid comprising a sequence encoding an immune cell stimulatory receptor agonist inserted downstream of the adenovirus fiber gene whereby expression of the encoded protein is driven by the adenovirus major late promoter. In a preferred embodiment, a heterologous nucleic acid comprising a sequence encoding an immune cell stimulatory receptor agonist is inserted in the E3 region of a replication-competent adenovirus backbone. The E3 region is nonessential for viral replication; however, the E3 proteins play a role in regulating host immune response. The replication-competent adenovirus can comprise a full or partial E3 deletion. For example, the replication-competent adenovirus can comprise deletions of one, two, three or more open reading frames (ORFs) in the E3 region and the heterologous nucleic acid inserted in its place. In one embodiment, the gpl9k and 6.7K genes are deleted and the heterologous nucleic acid is inserted into a gpl9k/6.7K deleted E3 region. In a related embodiment, the region between the *Bgl*II restriction enzyme sites at 78.3 and 85.8 map units of adenovirus type 5 genome may be deleted and the heterologous nucleic acid inserted into the deleted E3 region, as described in Bett et al., J. Virol., 67(10):5911-5922 (1993), the contents of which are incorporated herein by reference. In related aspects, the full E3 region is deleted from the replication-competent adenovirus backbone and the heterologous nucleic acid is inserted into a location containing the full E3 deletion. In a particularly preferred embodiment, the present invention provides a Delta-24 or Delta-24-RGD adenovirus comprising a heterologous nucleic acid inserted in place of a partially or completely deleted E3 region, wherein the heterologous nucleic acid comprises a sequence encoding an OX40 agonist, preferably OX40L and expression of the OX40 agonist is under the control of a non-adenoviral promoter such as a CMV promoter.

Certain embodiments are directed to methods of treating cancer comprising administering to a tumor a replication competent oncolytic virus (e.g. adenovirus) expressing one or more immune cell stimulatory receptor agonists as described above or a pharmaceutical composition comprising the replication-competent oncolytic virus. In certain aspects, the methods comprise administering to a tumor a Delta-24 adenovirus comprising a heterologous nucleic acid comprising a nucleic acid sequence encoding an immune cell stimulatory receptor agonist inserted into a non-essential region of the Delta-24 adenovirus backbone. In a preferred embodiment, part of the E3 region or all of the E3 region of the Delta-24 adenovirus genome is deleted and replaced with the heterlogous nucleic acid. In a particularly preferred embodiment, the present invention provides a method for treating cancer (e.g. glioma) in a human subject by administering to the subject a Delta-24-RGD adenovirus comprising a heterologous nucleic acid comprising a nucleic acid sequence encoding immune cell stimulatory receptor agonist (e.g. OX40L) into a non-essential region of the adenovirus backbone (e.g. a deleted E3 region). In some embodiments, the human subject exhibits a Th1 interluekine pattern. In other embodiments, the human subject exhibits a Th2 interleukine pattern. A subject is determined to exhibit a Th2 interleukine pattern if the subject has an IL-12/IL-4 ratio of less than about 20, less than about 15, or less than about 10. Subjects exhibiting a Th1 interleukine pattern will generally exhibit an IL-12/IL-4 ratio of greater than 20 and in some cases greater than 50, greater than 100 and even greater than 300. The IL-12/IL-4 ratio can be determined in the subject by obtaining a sample from the subject (e.g. a blood or serum sample), contacting the sample with antibodies against IL-12 and IL-4 and determining the amount of IL-12 and IL-4 in the sample as a function of the amount of binding of the antibodies to their respective antigens (e.g. by ELISA).

In related embodiments, one or more Th1 stimulating agents is co-administered with the replication competent oncolytic virus expressing one or more immune cell stimulatory receptor agonists as described above to treat cancer (e.g. glioblastoma) in a subject. In some embodiments, the subject has an IL-12/IL-4 ratio of less than about 20 (i.e. exhibits a Th2 interluekine pattern). In other embodiments, the subject has an IL-12/IL-4 ratio of greater than about 20 (i.e. exhibits a Th1 interleukine pattern). Th1 stimulating agents include, without limitation, (i) Th1 cytokines such as IL-12p70, IL-2 and IFN-γ, (ii) agents that increase production of Th1 cytokines such as REVLIMID (lenalidomide) (iii) agents that suppress regulatory T cells (e.g. alkylating agents such as temozolomide (4-methyl-5-oxo-2,3,4,6,8-pentazabicyclo[4.3.0]nona-2,7,9-triene-9-carboxamide), cyclophosphamide ((RS)-N,N-bis(2-chloroethyl)-1,3,2-oxazaphosphinan-2-amine 2-oxide), lomustine (CCNU; N-(2-chloroethyl)-N'-cyclohexyl-N-nitrosourea), bis-chloroethylnitrosourea (BCNU), melphalan hydrochloride (4 [bis(chloroethyl)amino]phenylalanine), busulfan (butane-1,4-diyl dimethanesulfonate), mechlorethamine (nitrogen mustard), chlorambucil, ifosfamide, streptozocin, dacarbazine (DTIC), thiotepa, altretamine (hexamethylmelamine), cisplatin, carboplatin, and oxalaplatin) and (iv) agents that stimulate cell mediated immune response (e.g. Ipilimumab, Tremelimumab, MDX-1106, MK-3475, AMP-224, Pidilizumab, and MDX-1105). Preferred Th1 stimulating agents to for co-administration with a replication competent oncolytic virus of the invention include IFN-γ (preferably recombinant) and temozolomide. The replication-competent oncolytic virus of the invention and a Th1 stimulating agent may be separately, concurrently or consecutively administered to a subject with cancer to treat the cancer. In one embodiment, the Th1 stimulating agent is administered to the subject and thereafter the replication-competent oncolytic virus is administered. In other related embodiments, a composition or kit is provided comprising (i) a Th1 stimulating agent and (ii) a replication-competent oncolytic adenovirus expressing one or more immune cell stimulatory receptor agonists as herein described, each in an amount effective to treat cancer in a subject in combination with the other. In a preferred embodiment, the composition or kit comprises (i) a Th1 stimulating agent selected from the group consisting of: recombinant IFN-γ, temozolomide, CCNU, BCNU, melphalan hydrochloride and busulfan and (ii) a replication-competent oncolytic adenovirus (e.g. Delta-24 or Delta-24-RGD) expressing an OX40 agonist (e.g. OX40L).

In certain embodiments, a replication-competent oncolytic virus (e.g. adenovirus) is provided that expresses a PD-L1 or PD-1 antagonist. In some embodiments, the replication-competent oncolytic virus express a PD-L1 or PD-1 antagonist in addition to expressing an immune cell stimulatory receptor agonist. In other embodiments, the replication-competent oncolytic virus expresses a PD-L1 or PD-1 antagonist but does not express an immune cell stimulatory receptor agonist. PD-L1 has been identified as a negative regulator of antitumor T cells and is expressed in up to 50% of human cancer. Binding of PD-L1 on tumor cells to PD-1 on activated effector T cells results in activation of PI3 kinase-signaling cascade which in turn blocks the production of cytotoxic mediators required for killing tumor cells. As used herein, a PD-L1 or PD-1 antagonist is a molecule that disrupts the interaction between PD-L1 and PD-1. In one aspect, the replication-competent oncolytic virus is an adenovirus that comprises heterologous nucleic acid encoding a PD-L1 or PD-1 antagonist inserted into a non-essential region of the adenovirus genome. In related aspects, the heterologous nucleic acid encodes an anti-PD-Ll antibody such as MPDL3280A, or an anti-PD-1 antibody such as nivolumab or lambrolizumab. In other embodiments, the heterologous nucleic acid encodes a PD-L1 or PD-1 antagonist such as those described in US Patent Application Publication Nos. 2009/0217401, 20110195068 and 20120251537 and US Patent No. 8,217,149, the contents of each which are incorporated herein by reference. In certain embodiments, a method for treating cancer (e.g. a glioma) in a human is provided comprising administering an effective amount of a replication-competent oncolytic virus expressing a PD-L1 and/or PD-1 antagonist. In a preferred embodiment, the replication-competent oncolytic virus is an adenovirus expressing a PD-L1 and/or PD-1 antagonist. In one preferred embodiment, the adenovirus is Delta-24 or Delta-24-RGD adenovirus.

In certain embodiments, the replication-competent oncolytic virus, in addition to expressing an immune cell stimulatory receptor agonist, also expresses one or more tumor antigens on its surface. In certain aspects, 1, 2, 3, 4, or 5 antigens are expressed on the surface of the virus, for example, by inserting nucleic acid encoding each antigen into a separate gene encoding an adenovirus surface protein. In a preferred embodiment, the tumor associated antigen(s) are EGFRvIII (epidermal growth factor receptor variant III) and/or NY-ESO-1 (New York oesophageal squamos cell carcinoma 1). The tumor antigens can be expressed as part of the capsid or fiber, or produced as exogenous proteins linked to autophagy-related proteins such as LC3 to increase the presentation of the exogenous protein during the adenoviral infection and replication. Targeting multiple antigens will help generate a consistent and effective immune response.

Tumor associated antigens (TAA) include, but are not limited to tumor associated antigens that have been identified as occurring in patients with brain cancers such as gliomas representative examples of which include: AIM2 (absent in melanoma 2), BMI1 (BMI1 polycomb ring finger oncogene), COX-2 (cyclooxygenase-2), TRP-1 (tyrosine related protein 2) TRP-2 (tyrosine related protein 2), GP100 (glycoprotein 100), EGFRvIII (epidermal growth factor receptor variant III), EZH2 (enhancer of zeste homolog 2), LICAM (human LI cell adhesion molecule), Livin, Livinβ, MRP-3 (multidrug resistance protein 3), Nestin, OLIG2 (oligodendrocyte transcription factor), SOX2 (SRY-related HMG-box 2), ART1 (antigen recognized by T cells 1), ART4 (antigen recognized by T cells 4), SART1 (squamous cell carcinoma antigen recognized by T cells 1), SART2, SART3, B-cyclin, b-catenin, Gli1 (glioma-associated oncogene homlog 1), Cav-1 (caveolin-1), cathepsin B, CD74 (cluster of Differentiation 74), E-cadherin (epithelial calcium-dependent adhesion), EphA2/Eck (EPH receptor A2/epithelial kinase), Fra-1/Fosl 1 (fos-related antigen 1), GAGE-1 (G antigen 1), Ganglioside/GD2, GnT-V, β1,6-N (acetylglucosaminyltransferase-V), Her2/neu (human epidermal growth factor receptor 2), Ki67 (nuclear proliferation-associated antigen of antibody Ki67), Ku70/80 (human Ku heterodimer proteins subunits), IL-13Ra2 (interleukin-13 receptor subunit alpha-2), MAGE-A (melanoma-associated antigen 1), MAGE-A3 (melanoma-associated antigen 3), NY-ESO-1 (New York oesophageal squamos cell carcinoma 1), MART-1 (melanoma antigen recognized by T cells), PROX1 (prospero homeobox protein 1), PSCA (prostate stem cell antigen), SOX10 (SRY-related HMG-box 10), SOX11, Survivin, UPAR (urokinase-type plasminogen activator receptor, and WT-1 (Wilms' tumor protein 1). The replication-competent oncolytic virus (e.g. adenovirus) may express the full length tumor associated antigen or an immunogenic peptide thereof.

In one aspect, the replication-competent oncolytic virus, in addition to expressing an immune cell stimulatory receptor agonist, also expresses EGFRvIII or an immunogenic peptide thereof on its surface. The sequence of EGFRvIII is described in U.S. Patent No. 6,455,498, the content of which is hereby incorporated by reference. Immunogenic EGFRvIII peptides include those described in U.S. Patent Application Publication No. 2009/0155282, the content of which is hereby incorporated by reference, particularly those at paragraph [0362] and Tables 4.1-4.3. Preferably, the oncolytic virus is an adenovirus and EGFRvIII or an immunogenic peptide thereof is inserted into the gene encoding the fiber protein, preferably in the HI loop. Nucleic acid encoding EGFRvIII or an immunogenic peptide thereof may be inserted into genes encoding one or more surface proteins of any adenovirus. The term "immunogenic EGFRvIII peptide" as used herein means a peptide of suitable length e.g. at least 10 or 12 amino acids and up to 15, 20, 25 or 30 amino acids or more which spans the mutated splice junction of the corresponding EGFRvIII protein, preferably human EGFRvIII. In a preferred embodiment, the nucleic acid inserted into an adenovirus surface protein encodes an 8-20 amino acid peptide consisting of, consisting essentially of, or comprising the sequence EKKGNYVV. In a particularly preferred embodiment, the EGFRvIII immunogenic peptide is LEEKKGNYVVT (SEQ ID NO: 4) and is inserted into the gene encoding the fiber protein, preferably in the HI loop. In other embodiments, nucleic acid encoding the entire EGFRvIII extracellular domain is inserted into a gene encoding a surface protein of the adenovirus.

In a related aspect, the replication-competent oncolytic virus, in addition to expressing an immune cell stimulatory receptor agonist, also expresses NY-ESO-1 (GenBank U87459.1) or an immunogenic peptide thereof (e.g. SLLMWITQCFLPVF) on its surface. Preferably, the replication-competent oncolytic virus is an adenovirus and the nucleic acid encoding NY-ESO-1 or an immunogenic peptide thereof is inserted into a gene encoding a surface protein, whereby the adenovirus expresses a chimeric surface protein comprising the NY-ESO-1 or an immunogenic peptide thereof. In one aspect, nucleic acid encoding NY-ESO-1 or an immunogenic peptide thereof is inserted into the hyper-variable region 5 of the gene encoding the hexon of the adenovirus.

Insertion of nucleic acids encoding the tumor antigens into adenovirus genes should be done "in frame" such that the virus expresses the tumor antigen on its surface.

Certain aspects do not require the complete resection of the tumor, which is a limiting factor in recruitment of patients in other approaches. Furthermore, certain aspects of the current methods and compositions have the potential to generate memory in the immune system and preventing or reducing the probability of tumor recurrence.

The term "replication competent" refers to any viral vector that is not deficient in any gene function required for viral replication in specific cells or tissues. The vector must be capable of replicating and being packaged, but might replicate only conditionally in specific cells or tissues. Replication competent adenoviral vectors of the present invention are engineered as described herein to reduce or eliminate their ability to replicate in normal cells while retaining their ability to replicate efficiently in specific tumor disease cell types. Typically, a replication competent adenovirus comprises enough of the E1, E2, and E4 regions that the adenovirus is capable of replicating and being packaged without the need for elements to be supplied in trans.

The term "therapeutic benefit" or "treatment" refers to anything that promotes or enhances the well-being of the subject with respect to the medical treatment of his/her condition, which includes treatment of pre-cancer, cancer, and hyperproliferative diseases. A list of nonexhaustive examples of this includes extension of the subject's life by any period of time, decrease or delay in the neoplastic development of the disease, decrease in hyperproliferation, reduction in tumor growth, delay of metastases, reduction in cancer cell or tumor cell proliferation rate, and a decrease in pain to the subject that can be attributed to the subject's condition.

A "T regulatory cell" or "regulatory T cell" refers to a cell that can inhibit a T cell response. Regulatory T cells express the transcription factor Foxp3, which is not upregulated upon T cell activation and discriminates regulatory T cells from activated effector cells. Regulatory T cells are identified by the cell surface markers CD25, CD45RB, CTLA4, and GITR. Regulatory T cell development is induced by myeloid suppressor cell activity. Several regulatory T cell subsets have been identified that have the ability to inhibit autoimmune and chronic inflammatory responses and to maintain immune tolerance in tumor- bearing hosts. These subsets include interleukin 10- (IL-10-) secreting T regulatory type 1 (TrI) cells, transforming growth factor-β-(TGF-β-) secreting T helper type 3 (Th3) cells, and "natural" CD4+/CD25+ Tregs (Tm) (Fehervari and Sakaguchi. J. Clin. Invest. 2004, 1 14: 1209- 1217; Chen et al. Science. 1994, 265: 1237-1240; Groux et al. Nature. 1997, 389: 737-742).

As used herein, an "agonist," e.g., an OX40 agonist, is a molecule which enhances the biological activity of its target, e.g., OX40. In certain aspects OX40 agonists, comprising, e.g., anti-OX40 antibodies or OX40 ligand compositions, substantially enhance the biological activity of OX40. Desirably, the biological activity is enhanced by 10%, 20%, 30%, 50%, 70%, 80%, 90%, 95%, or even 100%. In certain aspects, OX40 agonists as disclosed herein include OX40 binding molecules, e.g. binding polypeptides, anti-OX40 antibodies, OX40L, or fragments or derivatives of these molecules.

Other embodiments of the invention are discussed throughout this application. Any embodiment discussed with respect to one aspect of the invention applies to other aspects of the invention as well and vice versa. Each embodiment described herein is understood to be embodiments of the invention that are applicable to all aspects of the invention. It is contemplated that any embodiment discussed herein can be implemented with respect to any method or composition of the invention, and vice versa. Furthermore, compositions and kits of the invention can be used to achieve methods of the invention.

The use of the word "a" or "an" when used in conjunction with the term "comprising" in the claims and/or the specification may mean "one," but it is also consistent with the meaning of "one or more," "at least one," and "one or more than one."

Throughout this application, the term "about" is used to indicate that a value includes the standard deviation of error for the device or method being employed to determine the value.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages of the present invention will become apparent from the following detailed description. It should be understood, however, that the detailed description and the specific examples, while indicating specific embodiments of the invention, are given by way of illustration only, since various changes and modifications within the spirit and scope of the invention will become apparent to those skilled in the art from this detailed description.

### BRIEF DESCRIPTION OF THE DRAWINGS

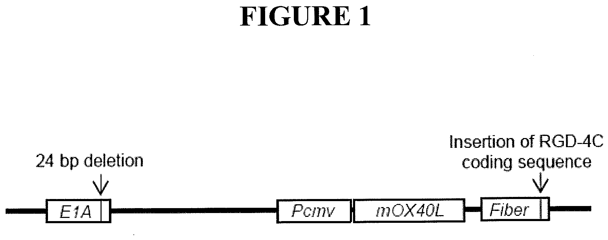

**FIG. 1****.** Construction of a novel adenovirus expressing the immune cell stimulatory receptor agonist OX40L. The genetic structure of Delta-24-RGD-OX40L is shown. Briefly, about 2.7 kb was removed from the non essential E3 region, from 78.3 to 85.8 map units, of Delta-24-RGD and a unique restriction enzyme site was introduced. An expression cassette for mouse OX40L cDNA driven by CMV promoter was then inserted into the deleted E3 region of the adenoviral genome utilizing the unique restriction site. In another construct, cDNA encoding mouse OX40L was inserted downstream of the fiber gene of the adenoviral genome and expression of OX40L was driven by the endogenous adenoviral late promoter.

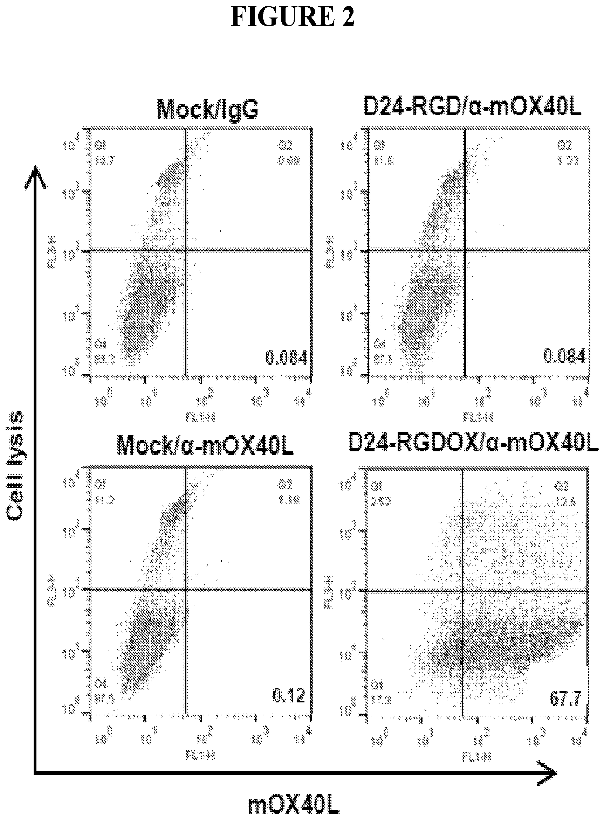

**FIG. 2****.** Expression of mouse OX4L (mOX40L) by Delta-24-RGD-OX40L (referred to as D24-RGDOX in the figure) on mouse glioma GL261 cells. GL261 cells were infected with the indicated viruses at 50 pfu/cell. 48 hours later, the cells were stained with α-mOX40L antibody (1:100 dilution). Cell membrane integrity was monitored with ethidium homodomer-1 staining (8 µM). The stained cells were analyzed with flow cytometry. The numbers at the lower right corners indicate percentage of cells expressing mOX40L.

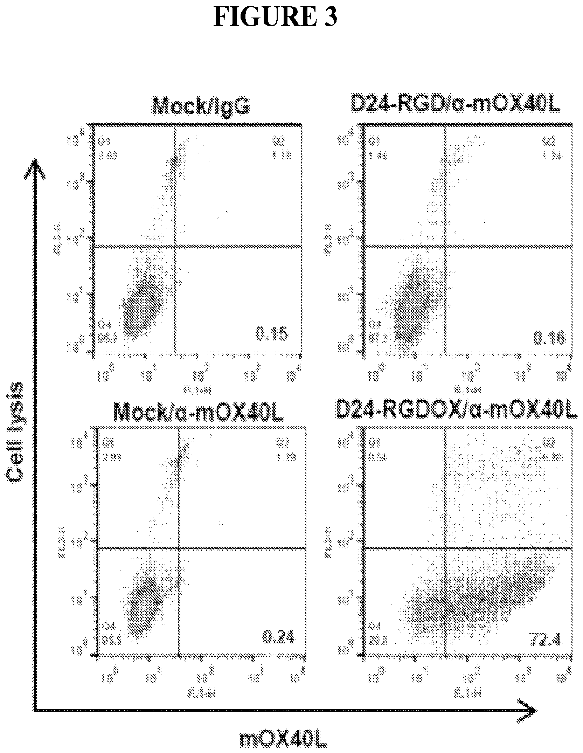

**FIG. 3****.** Expression of mouse OX40L (mOX40L) by D24-RGDOX on mouse melanoma B16 cells. Methods were the same as described for Figure 2.

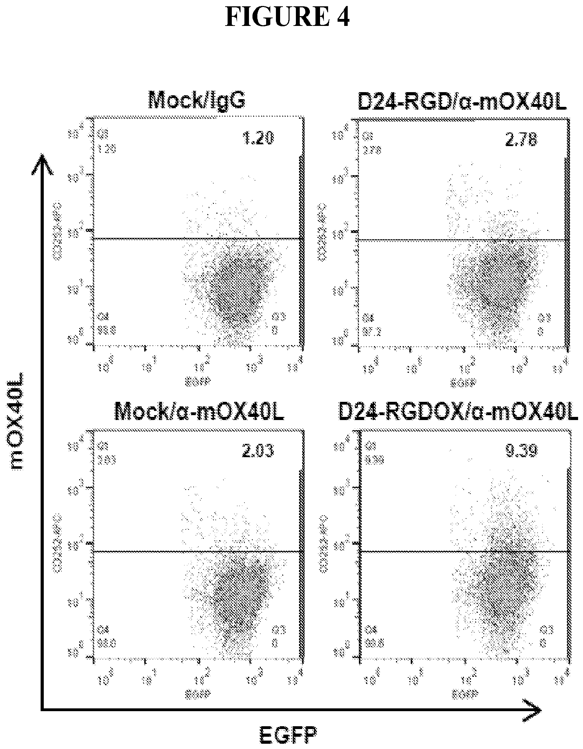

**FIG. 4****.** *In vivo* expression of mouse OX40L (mOX40L) by D24-RGDOX on xenograft cells. GL261-EGFP cells (5 x 10⁴ cells) were injected intracranially in C57BL/6 mice and 12 days later D24-RGDOX or D24-RGD were injected intratumorally (5 x 10⁷ pfu). 3 days after injection, the tumors were harvested and dissociated and the cells were stained with rat monoclonal α-mOX40L antibody (1:40 dilution). The stained cells were analyzed with flow cytometry. The numbers at the upper right corners indicate the percentage of tumor cells expressing mOX40L.

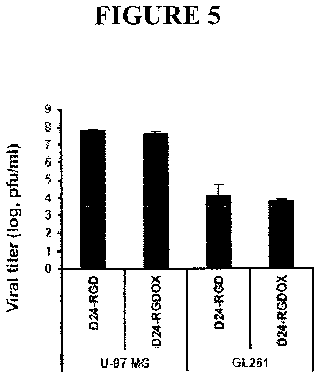

**FIG. 5****.** Replication of D24-RGD and D24-RGDOX in U-87 MG or GL261 cells. Cells were infected with the viruses at 10 pfu/cell. 48 hours after infection, infectious viral progeny were titered and final viral titers determined as pfu/ml.

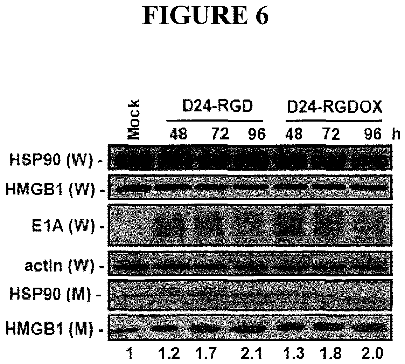

**FIG. 6****.** D24-RGD and D24-RGDOX induce release of HMGB1. GL261 cells were infected with the indicated viruses at 200 pfu/cell. 24 hour slater, the concentration of FBS was lowered from 10% to 2%. Culture medium (M) and whole cell lysates (W) were collected at the indicated time points and HSP90 and HMGB1 expression levels were analyzed with immunoblotting. The relative levels of HMGB1 in the medium are shown at the bottom of the panel.

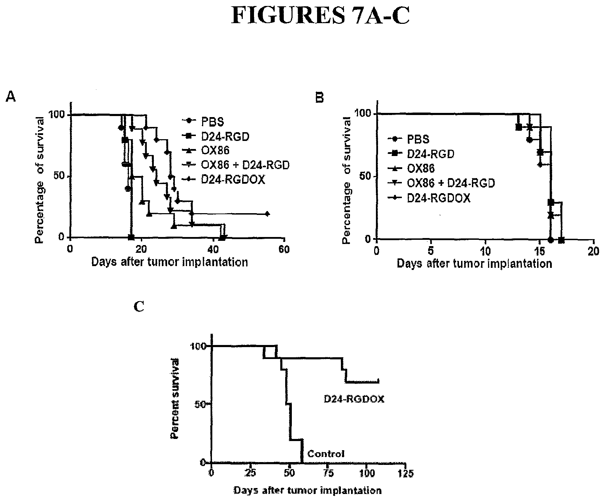

**FIGS. 7A-C****.** D24-RGDOX enhances anti-glioma immunity. Figure 7A: GL261 cells were implanted into the brain of C57BL/6 mice. Animals were randomly separated by groups (n=10) and treated (by intratumoral injection) with PBS, D24-RGDOX (5 x 10⁷ pfu), D24-RGD (5 x 10⁷ pfu), OX86 (a-mouse OX40 antibody) (25 µg), or D24-RGD in combination with OX86 (5 x 10⁷ pfu + 25 µg respectively). Animals showing generalized or local symptoms of disease were euthanized. Figure 7B: cells from a selected clone of GL261, characterized by a slower growing rate, were implanted into the brain of C57BL/6 mice. Survival studies were performed after treatment with control (PBS) or D24-RGDOX. Figure 7C: a similar experiment as in Figure 7A was performed in an immune deficient mouse model. In this model, D24-RGDOX did not increase the survival of intracranial glioma-bearing mice.

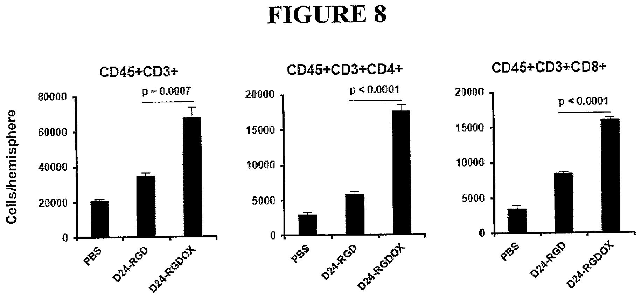

**FIG. 8****.** D24-RGDOX treatment results in higher recruitment of immune cells into the tumor bed than D24-RGD. PBS, D24-RGD or D24-RGDOX were administered intratumorally after GL261 cell intracranial implantation. On day 14 of the experiment, brains were collected and analyzed. Leukocytes from fresh tumor-containing hemispheres were isolated and analyzed with flow cytometry. P values are indicated (Student's t-test, double sided).

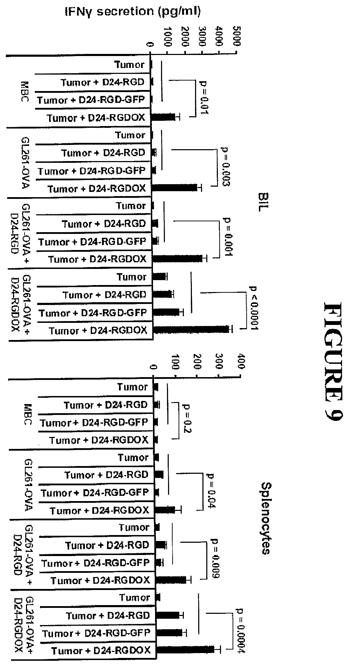

**FIG. 9****.** D24-RGDOX enhances immune response against tumor cells. Tumors were established as in Figure 8. D24-RGD or D24-RGDOX (5 x 10⁷ pfu) were injected intratumorally on days 6, 8 and 10 after tumor implantation. On day 14 after tumor implantation, splenocytes from mouse spleens (group of 5 mice) and brain infiltrated leukocytes (BILs) of each treatment were isolated. 2 x 10⁴ target cells (MBC (mouse brain cells), GL261-OVA, GL261-OVA + D24RGD or GL261-OVA + RGDOX) pre-fixed with 1% paraformaldehyde were incubated with 5 x 10⁴ BILs or 5 x 10⁵ splenocytes per well for 40 hours and the concentration of IFNγ in the supernatant assessed with standard ELISA.

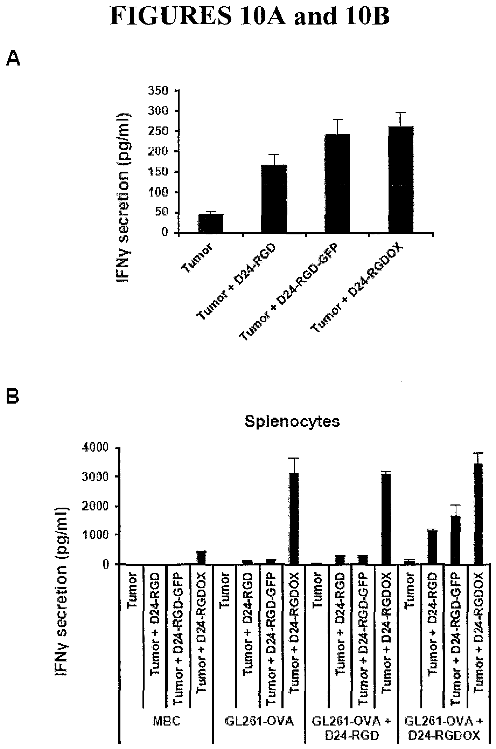

**FIGS. 10A-B****.** Activation of brain infiltrated lymphocytes and splenocytes. Figure 10A: The brain infiltrated lymphocytes were isolated from the mice from each treatment group on day 21 after tumor implantation and co-cultured with MBCs as described in Figure 9. Figure 10B: The splenocytes were isolated from the mice from each treatment group on day 21 after the tumor implantation and co-cultured with the indicated target cells as described in Figure 9. Forty hours later, the concentration of IFNγ in the supernatant was assessed with standard ELISA.

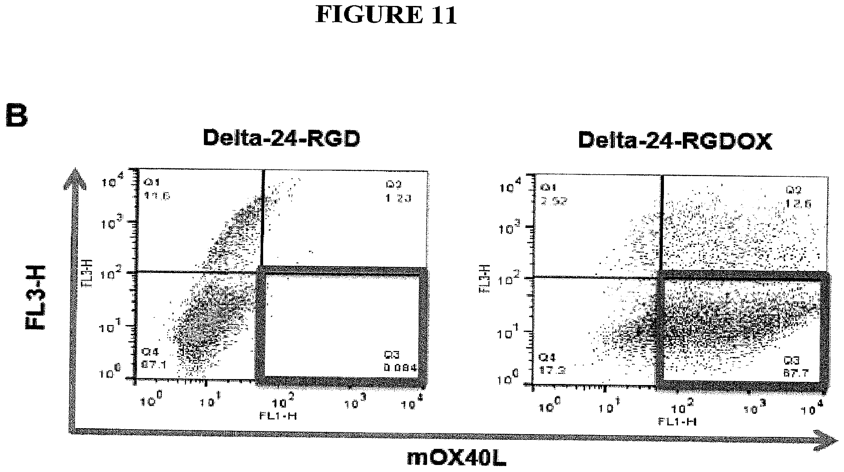

**FIG. 11****.** Graph demonstrating expression of OX40L in infected host cells following infection with Delta-24-RGD-OX40L (referred to as Delta-24-RGDOX in the figure). HeLa (human cervical epidermal adenocarcinoma) cells were infected with Delta-24-RGD-OX40L, constructed according to figure 1, at a multiplicity of infection (m.o.i.) of 50 pfu/cell. Briefly, viral stocks were diluted to the indicated m.o.i., added to cell monolayers (0.5 mL/60mm dish or 5 mL/100mm dish) and incubated at 37 C for 30 minutes with brief agitation every 5 minutes. After this, the necessary amount of culture medium was added and the cells were returned to the incubator for the prescribed time. 48 hours after infection with the virus, cells were stained with antibody against mOX40L and the percentage of cells expressing mOX40L analyzed by flow cytometry. Dead cells were excluded using EthD-1 staining (FL3-H). mOX40L positive cells are illustrated in the lower right quadrant. The images illustrate that cells infected with Delta-24-RGD-OX40L express OX40L.

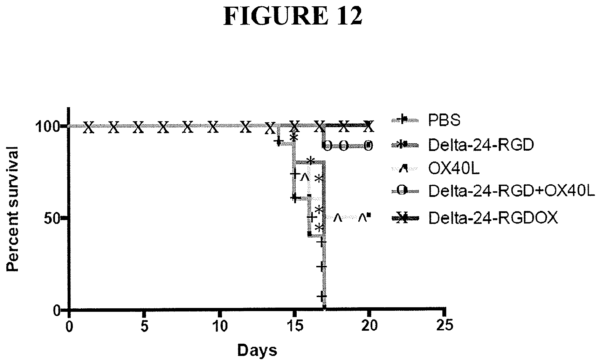

**FIG. 12****.** Graph showing enhanced survival of a mouse glioma model following treatment with Delta-24-RGD-OX40L (referred to as Delta-24-RGDOX in the figure). Data is presented as Kaplan-Meier curve of overall survival. Briefly, GL261 cells (5 x 10⁴) were implanted into the brain of C57BL/6 mice as described in Fueyo et al., J. Natl. Cancer Inst., 95:652-660 (2003). On days 3, 6 and 8 after tumor cell implantation, mice were randomly separated by groups (n=10) and intratumorally injected with 10 µL of solutions containing (1) Delta-24-RGD (10⁸ pfu/dose), (2) Delta-24-RGDOX (10⁸ pfu/dose) (3) OX40L antibody (25 µg/dose), (4) Delta-24-RGD in combination with OX40L antibody (10⁸ pfu/dose + 25mg/dose respectively) or (5) PBS as mock treatment.. Animals showing generalized or local symptoms of disease were euthanized. 100% of mice treated with Delta-24-RGDOX40L (Delta-24-RGDOX) were disease free after 20 days, whereas all mice treated with PBS (control) and all mice treated with Delta-24-RGD were euthanized by day 17. 50% of mice treated with OX-40L were disease free after 20 days. Importantly, Delta-24RGDOX40L treated mice exhibited enhanced survival relative to the group receiving separate treatments with Delta-24-RGD and OX40L antibody.

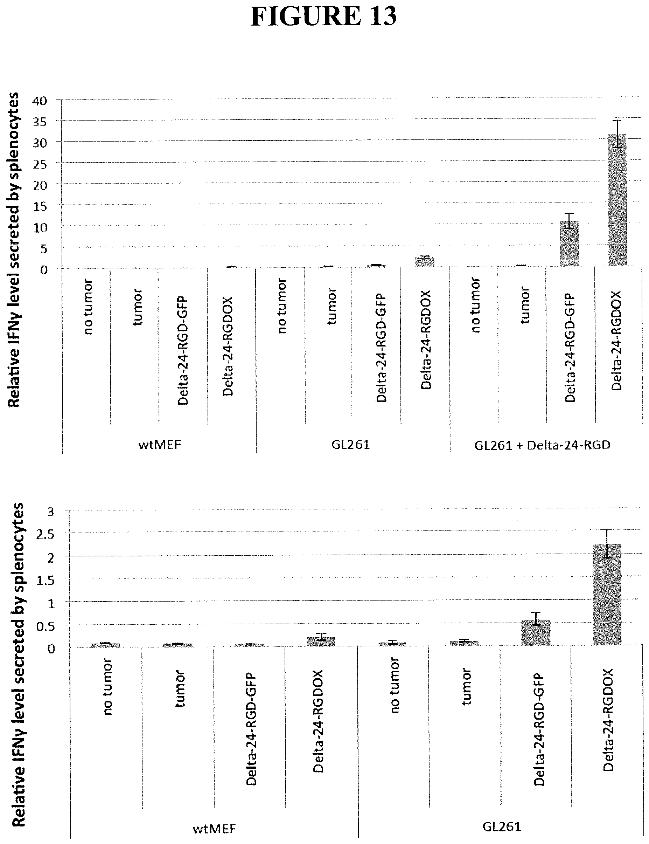

**FIG. 13****.** Graph showing enhanced TH1 response in a mouse glioma model following treatment with Delta-24-RGD-OX40L (referred to as Delta-24-RGDOX in the figure). GL261 cells were implanted into the brain of C57BL/6 mice. Mice were treated with intratumoral injections of Delta-24-GFP or Delta-24-RGD-OX40L (days 7, 9, 11 after tumor cell implantation). At day 14, mouse splenocytes were harvested from 3-5 mice per group and incubated with wild type mouse embryonic fibroblasts (wtMEF), GL261 or Delta-24-RGD-infected GL261 cells for 40 hours. The concentration of IFNγ secreted by splenocytes, as an indicator of splenocyte activation, was measured by ELISA. The bottom panel shows similar results depicted in the top panel for the first two groups of the experiment, using a different scale range. This data demonstrates that treatment with Delta-24-RGD-OX40L enhances the TH1 immune response to the tumor in the mouse model. Moreover, this data demonstrates that in addition to initiating anti-adenovirus immunity, glioma-bearing mice treated with Delta-24-RGD_OX40L develop a specific cellular response against infected and uninfected tumor cells. Thus, infection by Delta-24-RGDOX led to the development of anti-tumor immune response against cancer cells even if they had not been infected and suggests that by infecting a minority of tumor cells, Delta-24-RGDOX will elicit an immune response potentially capable of the eradication of the tumor.

### DESCRIPTION

Methods and compositions of the present invention include the construction and verification of oncolytic viruses (e.g. adenoviruses) comprising heterologous nucleic acid encoding an immune cell stimulatory receptor agonist that exhibit enhanced and even synergistic anti-tumor effects compared to the unmodified oncolytic virus (i.e. genetically similar or identical oncolytic virus not containing heterologous nucleic acid encoding an immune cell stimulatory receptor agonist) and the immune cell stimulatory receptor agonist when administered separately.

### I. Replication Competent Oncolytic Viruses

Replication-competent oncolytic viruses expressing one or more immune cell stimulatory receptor agonists according to the present invention include any naturally occurring (e.g. from a "field source") or modified replication-competent oncolytic virus. The oncolytic virus, in addition to expressing one or more immune cell stimulatory receptor agonists, may for example, be modified to increase selectivity of the virus for cancer cells.

Replication-competent oncolytic viruses according to the invention include, but are not limited to, oncolytic viruses that are a member in the family of myoviridae, siphoviridae, podpviridae, teciviridae, corticoviridae, plasmaviridae, lipothrixviridae, fuselloviridae, poxyiridae, iridoviridae, phycodnaviridae, baculoviridae, herpesviridae, adnoviridae, papovaviridae, polydnaviridae, inoviridae, microviridae, geminiviridae, circoviridae, parvoviridae, hepadnaviridae, retroviridae, cyctoviridae, reoviridae, birnaviridae, paramyxoviridae, rhabdoviridae, filoviridae, orthomyxoviridae, bunyaviridae, arenaviridae, leviviridae, picornaviridae, sequiviridae, comoviridae, potyviridae, caliciviridae, astroviridae, nodaviridae, tetraviridae, tombusviridae, coronaviridae, glaviviridae, togaviridae, and barnaviridae.

Particular examples of replication-competent oncolytic viruses for use in the practice of the invention include adenovirus, retrovirus, reovirus, rhabdovirus, Newcastle Disease virus (NDV), polyoma virus, vaccinia virus, herpes simplex virus, picornavirus, coxsackie virus and parvovirus

In one embodiment, the replication-competent oncolytic virus is a rhabdovirus selected from a vesicular stomatitis virus (VSV) and a Maraba strain, optionally modified to increase cancer selectivity. Such modifications include, but are not limited to, mutations in the matrix (M) gene that render the virus susceptible to a host IFN response.

In another embodiment, the replication-competent oncolytic virus is a vaccinia virus, non-limiting examples of which include Western Reserve, Wyeth, and Copenhagen strains optionally modified to increase cancer selectivity. Such modifications include, but are not limited to: non-functional thymidine kinase gene, non-functional vaccinia growth factor gene, and non-functional type 1 interferon-binding gene.

In another aspect, the replication competent oncolytic virus is selected from a herpes simplex virus (HSV) virus (such as HSV-1 or HSV1716) and a Newcastle disease virus (NDV).

Adenoviruses are particularly preferred replication-competent oncolytic viruses.

Adenovirus (Ad) is a large (∼36 kb) DNA virus that infects humans, but which display a broad host range. Physically, adenovirus is an icosahedral virus containing a double-stranded, linear DNA genome. There are approximately 50 serotypes of human adenovirus, which are divided into six families based on molecular, immunological, and functional criteria. By adulthood, virtually every human has been infected with the more common adenovirus serotypes, the major effect being cold-like symptoms.

Adenoviral infection of host cells results in adenoviral DNA being maintained episomally, which reduces the potential genotoxicity associated with integrating vectors. Also, adenoviruses are structurally stable, and no genome rearrangement has been detected after extensive amplification. Adenovirus can infect virtually most epithelial cells regardless of their cell cycle stage. So far, adenoviral infection appears to be linked only to mild disease such as acute respiratory disease in humans

Members of any of the 57 human adenovirus serotypes (HAdV-1 to 57) may incorporate heterologous nucleic acid encoding an immune cell stimulatory receptor agonist according to the invention. Human Ad5 is well characterized genetically and biochemically (GenBank M73260; AC_000008). Thus, in a preferred embodiment, the oncolytic adenovirus is a replication competent Ad5 serotype or a hybrid serotype comprising an Ad5 component. The adenovirus may be a wild type strain but is preferably genetically modified to enhance tumor selectivity, for example by attenuating the ability of the virus to replicate within normal quiescent cells without affecting the ability of the virus to replicate in tumor cells. Non-limiting examples of replication competent oncolytic adenoviruses encompassed by the present invention include Delta-24, Delta-24-RGD, ICOVIR-5, ICOVIR-7, ONYX-015, ColoAd1, H101 and AD5/3-D24-GMCSF. Onyx-015 is a hybrid of virus serotype Ad2 and Ad5 with deletions in the E1B-55K and E3B regions to enhance cancer selectivity. H101 is a modified version of Onyx-015. ICOVIR-5 and ICOVIR-7 comprise an Rb-binding site deletion of E1A and a replacement of the E1A promoter by an E2F promoter. ColoAd1 is a chimeric Add11p/Ad3 serotype. AD5/3-D24-GMCSF (CGTG-102) is a serotype 5/3 capsid-modified adenovirus encoding GM-CSF (the Ad5 capsid protein knob is replaced with a knob domain from serotype 3).

In one particularly preferred embodiment, the replication competent oncolytic adenovirus is Delta-24 or Delta-24-RGD. Delta-24 is described in U.S. Patent Application Publication Nos. 20030138405, and 20060147420, each of which are incorporated herein by reference. The Delta-24 adenovirus is derived from adenovirus type 5 (Ad-5) and contains a 24-base-pair deletion within the CR2 portion of the E1A gene that encompasses the area responsible for binding Rb protein (nucleotides 923-946) corresponding to amino acids 122-129 in the encoded E1A protein (Fueyo J et al., Oncogene, 19:2-12 (2000)). Delta-24-RGD further comprises an insertion of the RGD-4C sequence (which binds strongly to αvβ3 and α vβ5 integrins) into the H1 loop of the fiber knob protein (Pasqualini R. et al., Nat Biotechnol, 15:542-546 (1997)). The E1A deletion increases the selectivity of the virus for cancer cells; the RGD-4C sequence increases the infectivity of the virus in gliomas.

Oncolytic adenoviruses injected into a tumor induce cell death and release of new adenovirus progeny that, by infecting the neighbor cells, generates a treatment wave that, if not halted, may lead to the total destruction of the tumor. Significant antitumor effects of Delta-24 have been shown in cell culture systems and in malignant glioma xenograft models. Delta-24-RGD has shown surprising anti-tumor effects in a Phase 1 clinical trial and is currently the subject of additional clinical trials. Although lysis of tumor cells is the main anti-cancer mechanism proposed for Delta-24-RGD oncolytic adenovirus, data from the Phase 1 clinical trial in patients with recurrent glioma and other observations indicate that the direct oncolytic effect may be enhanced by the adenovirus-mediated trigger of anti-tumor immune response. Thus, approximately 10% of patients treated with Delta-24-RGD showed an infiltration of the tumor by immune cells that in certain cases is quite massive. In these cases, representing a small minority of those treated, a Th1-predominant immune response was observed that appears to correlate with optimum anti-tumor response. Aspects of the current invention are directed at enhancing this anti-tumor efficacy in the majority of patients. The replication-competent oncolytic adenovirus of the invention is designed to accomplish this by (i) enhancing the Th1 immune response against both adenoviral and tumor antigens and (2) reversing the immune suppressive environment of the tumor. Administration of oncolytic adenovirus of the invention leads to the activation of the population of lymphocytes that recognize cancer cells with or without virus infection and accordingly provides an enhanced and prolonged antitumor effect that persists even after the virus is eradicated. Moreover, activation of immune cell stimulatory receptors such as OX40 leads to a decrease in the number and activation status of T regulatory cells which play a role in maintaining the immune suppressed environment of tumors. Oncolytic adenovirus of the invention provides a significant advantage compared to separately administering the adenovirus and the immune cell stimulatory receptor agonist by localizing the agonist to the site of the tumor thereby reducing unwanted side-effects accompanying systemic administration of the agonist.

The infectious cycle of the adenovirus takes place in 2 steps: the early phase which precedes initiation of the replication of the adenoviral genome, and which permits production of the regulatory proteins and proteins involved in the replication and transcription of the viral DNA, and the late phase which leads to the synthesis of the structural proteins. The early genes are distributed in 4 regions that are dispersed in the adenoviral genome, designated E1 to E4 (E denotes "early"). The early regions comprise at least-six transcription units, each of which possesses its own promoter. The expression of the early genes is itself regulated, some genes being expressed before others. Three regions, E1, E2, and E4 are essential to replication of the virus. Thus, if an adenovirus is defective for one of these functions this protein will have to be supplied in trans, or the virus cannot replicate.

The E1 early region is located at the 5' end of the adenoviral genome, and contains 2 viral transcription units, E1A and E1B. This region encodes proteins that participate very early in the viral cycle and are essential to the expression of almost all the other genes of the adenovirus. In particular, the E1A transcription unit codes for a protein that transactivates the transcription of the other viral genes, inducing transcription from the promoters of the E1B, E2A, E2B, E3, E4 regions and the late genes. Typically, exogenous sequences are integrated in place of all or part of the E3 region

The adenovirus enters the permissive host cell via a cell surface receptor, and it is then internalized. The viral DNA associated with certain viral proteins needed for the first steps of the replication cycle enters the nucleus of the infected cells, where transcription is initiated. Replication of the adenoviral DNA takes place in the nucleus of the infected cells and does not require cell replication. New viral particles or virions are assembled after which they are released from the infected cells, and can infect other permissive cells.

The adenovirus is an attractive delivery system. Embodiments of the invention can utilize a suspension cell process with average yields of 1×10¹⁶ viral particles per batch. The process can be free of or essentially free of protein, serum, and animal derived components making it suitable for a broad range of both prophylactic and therapeutic vaccine products.

Several factors favor the use of oncolytic adenoviruses for the treatment of brain tumors. First, gliomas are typically localized, and therefore an efficient local approach should be enough to cure the disease. Second, gliomas harbor several populations of cells expressing different genetic abnormalities. Thus, the spectrum of tumors sensitive to the transfer of a single gene to cancer cells may be limited. Third, replication competent adenoviruses can infect and destroy cancer cells that are arrested in G0. Since gliomas invariably include non-cycling cells, this property is important. Finally, the p16-Rb pathway is abnormal in the majority of gliomas, thus making Delta-24 adenovirus particularly effective for treating these tumors, although the loss of the retinoblastoma tumor suppressor gene function has been associated with the causes of various types of tumors and is not limited to treatment of gliomas.

If an adenovirus has been mutated so that it is conditionally replicative (replication-competent under certain conditions), a helper cell may be required for viral replication. When required, helper cell lines may be derived from human cells such as human embryonic kidney cells, muscle cells, hematopoietic cells or other human embryonic mesenchymal or epithelial cells. Alternatively, the helper cells may be derived from the cells of other mammalian species that are permissive for human adenovirus. Such cells include, for example Vero cells or other monkey embryonic mesenchymal or epithelial cells. In certain aspects a helper cell line is 293. Various methods of culturing host and helper cells may be found in the art, for example Racher et al., 1995.

In certain aspects, the oncolytic adenovirus is replication-competent in cells with a mutant Rb pathway. After transfection, adenoviral plaques are isolated from the agarose-overlaid cells and the viral particles are expanded for analysis. For detailed protocols the skilled artisan is referred to Graham and Prevac, 1991.

Alternative technologies for the generation of adenovirus vectors include utilization of the bacterial artificial chromosome (BAC) system, in vivo bacterial recombination in a recA+bacterial strain utilizing two plasmids containing complementary adenoviral sequences, and the yeast artificial chromosome (YAC) system (PCT publications 95/27071 and 96/33280, which are incorporated herein by reference).

Adenovirus is easy to grow and manipulate and exhibits broad host range *in vitro* and *in vivo.* This group of viruses can be obtained in high titers (e.g., greater than 10⁹ plaque forming units (pfu) per ml), and they are highly infective. The life cycle of adenovirus does not require integration into the host cell genome.

Modifications of oncolytic adenovirus described herein may be made to improve the ability of the oncolytic adenovirus to treat cancer. Such modifications of an oncolytic adenovirus have been described by Jiang et al. (Curr Gene Ther. 2009 Oct 9(5):422-427), see also U.S. Patent Application No. 20060147420, each of which are incorporated herein by reference.

The absence or the presence of low levels of the coxsackievirus and adenovirus receptor (CAR) on several tumor types can limit the efficacy of the oncolytic adenovirus. Various peptide motifs may be added to the fiber knob, for instance an RGD motif (RGD sequences mimic the normal ligands of cell surface integrins), Tat motif, polylysine motif, NGR motif, CTT motif, CNGRL motif, CPRECES motif or a strept-tag motif (Rouslahti and Rajotte, 2000). A motif can be inserted into the HI loop of the adenovirus fiber protein. Modifying the capsid allows CAR independent target cell infection. This allows higher replication, more efficient infection, and increased lysis of tumor cells (Suzuki et al., 2001, incorporated herein by reference). Peptide sequences that bind specific human glioma receptors such as EGFR or uPR may also be added. Specific receptors found exclusively or preferentially on the surface of cancer cells may be used as a target for adenoviral binding and infection, such as EGFRvIII.

### II. Expression Cassettes

In certain embodiments of the present invention, the methods set forth herein involve nucleic acid sequences encoding an immune cell stimulatory receptor agonist wherein the nucleic acid is comprised in an "expression cassette." The term "expression cassette" is meant to include any type of genetic construct containing a nucleic acid coding for a gene product in which part or all of the nucleic acid encoding sequence is capable of being transcribed.

Promoters and Enhancers - In order for the expression cassette to effect expression of a transcript, the nucleic acid encoding gene will be under the transcriptional control of a promoter. A "promoter" is a control sequence that is a region of a nucleic acid sequence at which initiation and rate of transcription are controlled. The phrases "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence.

Any promoter known to those of ordinary skill in the art that would be active in a cell in a subject is contemplated as a promoter that can be applied in the methods and compositions of the present invention. One of ordinary skill in the art would be familiar with the numerous types of promoters that can be applied in the present methods and compositions. In certain embodiments, for example, the promoter is a constitutive promoter, an inducible promoter, or a repressible promoter. The promoter can also be a tissue selective promoter. A tissue selective promoter is defined herein to refer to any promoter that is relatively more active in certain tissue types compared to other tissue types. Examples of promoters include the CMV promoter.

The promoter will be one that is active in a cell and expression from the promoter results in the presentation of an antigenic determinant to a subject's immune system. For instance, where the cell is an epithelial cell the promoter used in the embodiment will be one having activity in that particular cell type.

A promoter may be one naturally associated with a gene or sequence, as may be obtained by isolating the 5'-non-coding sequences located upstream of the coding segment and/or exon. Such a promoter can be referred to as "endogenous." Similarly, an enhancer may be one naturally associated with a nucleic acid sequence, located either downstream or upstream of that sequence. Alternatively, certain advantages will be gained by positioning the coding nucleic acid segment under the control of a recombinant or heterologous promoter, which refers to a promoter that is not normally associated with a nucleic acid sequence in its natural environment. A recombinant or heterologous enhancer refers also to an enhancer not normally associated with a nucleic acid sequence in its natural environment. Such promoters or enhancers may include promoters or enhancers of other genes, and promoters or enhancers isolated from any other prokaryotic, viral, or eukaryotic cell, and promoters or enhancers not "naturally occurring," i.e., containing different elements of different transcriptional regulatory regions, and/or mutations that alter expression. In addition to producing nucleic acid sequences of promoters and enhancers synthetically, sequences may be produced using recombinant cloning and/or nucleic acid amplification technology, including PCR™ (see U.S. Pat. Nos. 4,683,202 and 5,928,906, each incorporated herein by reference).

Naturally, it will be important to employ a promoter and/or enhancer that effectively directs the expression of the DNA segment in the cell type, organelle, and organism chosen for expression. Those of skill in the art of molecular biology generally understand the use of promoters, enhancers, and cell type combinations for protein expression, for example, see Sambrook et al. (2001), incorporated herein by reference. The promoter may be heterologous or endogenous.

The particular promoter that is employed to control the expression of the nucleic acid of interest is not believed to be critical, so long as it is capable of expressing the polynucleotide in the targeted cell at sufficient levels. Thus, where a human cell is targeted, it is preferable to position the polynucleotide coding region adjacent to and under the control of a promoter that is capable of being expressed in a human cell. Generally speaking, such a promoter might include either a human or viral promoter.

In various embodiments, the human cytomegalovirus (CMV) immediate early gene promoter, the SV40 early promoter and the Rous sarcoma virus long terminal repeat can be used. The use of other viral or mammalian cellular or bacterial phage promoters, which are well-known in the art to achieve expression of polynucleotides, is contemplated as well, provided that the levels of expression are sufficient to produce an immune response.

Additional examples of promoters/elements that may be employed, in the context of the present invention include the following, which is not intended to be exhaustive of all the possible promoter and enhancer elements, but, merely, to be exemplary thereof: Immunoglobulin Heavy Chain; Immunoglobulin Light Chain; T Cell Receptor; HLA DQ α and/or DQ β; β Interferon; Interleukin-2; Interleukin-2 Receptor; MHC Class II; MHC Class II HLA-DRα; β-Actin; Muscle Creatine Kinase (MCK); Prealbumin (Transthyretin); Elastase I; Metallothionein (MTII); Collagenase; Albumin; α-Fetoprotein; t-Globin; β-Globin; c-fos; c-HA-ras; Insulin; Neural Cell Adhesion Molecule (NCAM); α1-Antitrypsin; H2B (TH2B) Histone; Mouse and/or Type I Collagen; Glucose-Regulated Proteins (GRP94 and GRP78); Rat Growth Hormone; Human Serum Amyloid A (SAA); Troponin I (TN I); Platelet-Derived Growth Factor (PDGF); Duchenne Muscular Dystrophy; SV40; Polyoma; Retroviruses; Papilloma Virus; Hepatitis B Virus; Human Immunodeficiency Virus; Cytomegalovirus (CMV); and Gibbon Ape Leukemia Virus.

Enhancers were originally detected as genetic elements that increased transcription from a promoter located at a distant position on the same molecule of DNA. The basic distinction between enhancers and promoters is operational. An enhancer region as a whole must be able to stimulate transcription at a distance; this need not be true of a promoter region or its component elements. On the other hand, a promoter must have one or more elements that direct initiation of RNA synthesis at a particular site and in a particular orientation, whereas enhancers lack these specificities. Promoters and enhancers are often overlapping and contiguous, often seeming to have very similar modular organization. Additionally, any promoter/enhancer combination (as per the Eukaryotic Promoter Data Base EPDB) could also be used to drive expression of a gene. Further selection of a promoter that is regulated in response to specific physiologic signals can permit inducible expression of a construct. For example, with the polynucleotide under the control of the human PAI-1 promoter, expression is inducible by tumor necrosis factor. Examples of inducible elements, which are regions of a nucleic acid sequence that can be activated in response to a specific stimulus include (Element/Inducer): MT II/Phorbol Ester (TFA) or Heavy metals; MMTV (mouse mammary tumor virus)/Glucocorticoids; P-Interferon/poly(rI)x or poly(rc); Adenovirus 5 E2/E1A; Collagenase/Phorbol Ester (TPA); Stromelysin/Phorbol Ester (TPA); SV40/Phorbol Ester (TPA); Murine MX Gene/Interferon, Newcastle Disease Virus; GRP78 Gene/A23187; α-2-Macroglobulin/IL-6; Vimentin/Serum; MHC Class I Gene H-2κb/Interferon; HSP70/E1A, SV40 Large T Antigen; Proliferin/Phorbol Ester-TPA; Tumor Necrosis Factor/PMA; and Thyroid Stimulating Hormone a Gene/Thyroid Hormone.

Initiation Signals - A specific initiation signal also may be required for efficient translation of coding sequences. These signals include the ATG initiation codon or adjacent sequences. Exogenous translational control signals, including the ATG initiation codon, may need to be provided. One of ordinary skill in the art would readily be capable of determining this and providing the necessary signals.

IRES - In certain embodiments of the invention, the use of internal ribosome entry sites (IRES) elements are used to create multigene, or polycistronic, messages. IRES elements are able to bypass the ribosome scanning model of 5' methylated Cap dependent translation and begin translation at internal sites. IRES elements from two members of the picornavirus family (polio and encephalomyocarditis) have been described, as well an IRES from a mammalian message. IRES elements can be linked to heterologous open reading frames. Multiple open reading frames can be transcribed together, each separated by an IRES, creating polycistronic messages (see U.S. Pat. Nos. 5,925,565 and 5,935,819).

Multiple Cloning Sites - Expression cassettes can include a multiple cloning site (MCS), which is a nucleic acid region that contains multiple restriction enzyme sites, any of which can be used in conjunction with standard recombinant technology to digest the vector.

Polyadenylation Signals - In expression, one will typically include a polyadenylation signal to effect proper polyadenylation of the transcript. The nature of the polyadenylation signal is not believed to be crucial to the successful practice of the invention, and/or any such sequence may be employed. Preferred embodiments include the SV40 polyadenylation signal and/or the bovine growth hormone polyadenylation signal, convenient and/or known to function well in various target cells. Also contemplated as an element of the expression cassette is a transcriptional termination site. These elements can serve to enhance message levels and/or to minimize read through from the cassette into other sequences.

Other Expression Cassette Components - In certain embodiments of the invention, cells infected by the adenoviral vector may be identified in vitro by including a reporter gene in the expression vector. Generally, a selectable reporter is one that confers a property that allows for selection. A positive selectable reporter is one in which the presence of the reporter gene allows for its selection, while a negative selectable reporter is one in which its presence prevents its selection. An example of a positive selectable marker is a drug resistance marker (genes that confer resistance to neomycin, puromycin, hygromycin, DHFR, GPT, zeocin and histidinol). Other types of reporters include screenable reporters such as GFP.

Embodiments of the invention can use current adenoviral platform technologies in the preparation of an adenoviral nucleic acid comprising a heterologous nucleic acid segment that encodes a tumor associated antigen. Aspects of the adenoviral vaccine construction include inserting genetic material into an adenoviral vector and confirming the construct through characterization and sequencing of the nucleic acid, virus and virus product. The adenoviral vaccine is then put through a series of feasibilities studies designed to assess scalability.

### III. Cancer

The methods of the present invention may be used to treat cancers. Specific examples of cancer types include but are not limited to glioma, melanoma, metastases, adenocarcinoma, thyoma, lymphoma, sarcoma, lung cancer, liver cancer, colon cancer, non-Hodgkins lymphoma, Hodgkins lymphoma, leukemias, uterine cancer, breast cancer, prostate cancer, ovarian cancer, cervical cancer, bladder cancer, kidney cancer, pancreatic cancer and the like.

The term "glioma" refers to a tumor originating in the neuroglia of the brain or spinal cord. Gliomas are derived from the glial cell types such as astrocytes and oligodendrocytes, thus gliomas include astrocytomas and oligodendrogliomas, as well as anaplastic gliomas, glioblastomas, and ependymomas. Astrocytomas and ependymomas can occur in all areas of the brain and spinal cord in both children and adults. Oligodendrogliomas typically occur in the cerebral hemispheres of adults. Gliomas account for 75% of brain tumors in pediatrics and 45% of brain tumors in adults. Other brain tumors are meningiomas, ependymomas, pineal region tumors, choroid plexus tumors, neuroepithelial tumors, embryonal tumors, peripheral neuroblastic tumors, tumors of cranial nerves, tumors of the hemopoietic system, germ cell tumors, and tumors of the stellar region. The methods of the present invention may be used to treat any cancer of the brain.

The term melanoma includes, but is not limited to, melanomas, metastatic melanomas, melanomas derived from either melanocytes or melanocytes related nevus cells, melanocarcinomas, melanoepitheliomas, melanosarcomas, melanoma in situ, superficial spreading melanoma, nodular melanoma, lentigo maligna melanoma, acral lentiginous melanoma, invasive melanoma or familial atypical mole and melanoma (FAM-M) syndrome. Such melanomas in mammals may be caused by, chromosomal abnormalities, degenerative growth and developmental disorders, mitogenic agents, ultraviolet radiation (UV), viral infections, inappropriate tissue expression of a gene, alterations in expression of a gene, and presentation on a cell, or carcinogenic agents. The aforementioned cancers can be assessed or treated by methods of the present invention. In the case of cancer, a gene encoding an antigen associated with the cancer (e.g. a tumor associated antigen (TAA)) may be incorporated into the recombinant virus genome or portion thereof along with nucleic acid encoding one or more immune cell stimulatory receptor agonist molecules. The antigen associated with the cancer may be expressed on the surface of a cancer cell, may be secreted or may be an internal antigen.

### IV. Pharmaceutical Compositions

The present invention also provides a pharmaceutical composition comprising any composition of the present invention, and a pharmaceutically acceptable carrier. The present invention also provides a vaccine composition comprising any composition of the present invention. The vaccine composition may further comprise at least one adjuvant.

The present invention also provides a method of stimulating an anti-tumor immune response in a subject, comprising administering to a subject a composition of the present invention.

According to the present invention, an adenovirus expressing one or more immune cell stimulatory receptor agonists and optionally one or more tumor associated antigens is administered to a subject to induce an immune response for therapeutic or prophylatic purposes. Thus, in certain embodiments, the expression construct is formulated in a composition that is suitable for this purpose. The phrases "pharmaceutically" or "pharmacologically acceptable" refer to compositions that do not produce adverse, allergic, or other untoward reactions when administered to an animal or a human. As used herein, "pharmaceutically acceptable carrier" includes any and all solvents, carriers, dispersion media, coatings, antibacterial and antifungal agents, isotonic and absorption delaying agents, and the like. The use of such media and agents for pharmaceutically active substances is well known in the art. Except insofar as any conventional media or agent is incompatible with the expression constructs of the present invention, its use in therapeutic compositions is contemplated. Supplementary active ingredients also can be incorporated into the compositions. For example, the supplementary active ingredient may be an additional immunogenic agent.

The pharmaceutical forms suitable for injectable use include sterile aqueous solutions or dispersions and sterile powders for the extemporaneous preparation of sterile injectable solutions or dispersions. In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. If needed, various antibacterial an antifungal agents can be used, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating compounds in the required amount in the appropriate solvent with various of the other ingredients enumerated above, as required, followed by filter sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of preparation are vacuum-drying and freeze-drying techniques which yield a powder of the active ingredient plus any additional desired ingredient from a previously sterile-filtered solution thereof.

Upon formulation, solutions will be administered in a manner compatible with the dosage formulation and in such amount as is therapeutically or prophylactically effective. For parenteral administration in an aqueous solution, the solution should be suitably buffered if necessary and the liquid diluent first rendered isotonic with sufficient saline or glucose. These particular aqueous solutions are especially suitable for intravascular and intratumoral administration. In this connection, sterile aqueous media, which can be employed will be known to those of skill in the art in light of the present disclosure.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by the FDA.

Dosage - An effective amount of the therapeutic or preventive agent is determined based on the intended goal, for example stimulation of an immune response against a tumor. Those of skill in the art are well aware of how to apply gene delivery in vivo and ex vivo situations. For viral vectors, one generally will prepare a viral vector stock. Depending on the kind of virus and the titer attainable, one will deliver at least about, at most about, or about 1×10⁴, 1×10⁵, 1×10⁶, 1×10⁷, 1×10⁸, 1×10⁹, 1×10¹¹, 1×10¹¹ or 1×10¹² infectious particles, or any value or range there between, to a subject. In other aspects, adenoviruses according to the invention may be administered in a single administration or multiple administrations. The virus may be administered at dosage of 1 x 10⁵ plaque forming units (PFU), 5 x 10⁵ PFU, at least 1 x 10⁶ PFU, 5 x 10⁶ or about 5 x 10⁶ PFU, 1 x 10⁷, at least 1 x 10⁷ PFU, 1 x 10⁸ or about 1 x 10⁸ PFU, at least 1 x 10⁸ PFU, about or at least 5 x 10⁸ PFU, 1 x 10⁹ or at least 1 x 10⁹ PFU, 5 x 10⁹ or at least 5 x 10⁹ PFU, 1 x 10¹⁰ PFU or at least 1 x 10¹⁰ PFU, 5 x 10¹⁰ or at least 5 x 10¹⁰ PFU, 1 x 10¹¹ or at least 1 x10¹¹, 1 x 10¹² or at least 1 x 10¹², 1 x 10¹³ or at least 1 x 10¹³ PFU. For example, the virus may be administered at a dosage of between about 10⁷-10¹³ PFU, between about 10¹-10¹³ PFU, between about 10⁹-10¹² PFU, or between about 10⁸-10¹² PFU.

Replication-competent oncolytic viruses according to the invention may be administered locally or systemically. For example, without limitation, oncolytic viruses according to the invention can be administered intravascularly (intraarterially or intravenously), intratumorally, intramuscularly, intradermally, intraperitoneally, subcutaneously, orally, parenterally, intranasally, intratracheally, percutaneously, intraspinally, ocularly, or intracranially. In preferred embodiments, an adenovirus of the invention is administered intravascularly or intratumorally.

Replication-competent oncolytic viruses according to the invention may also be administered in a cellular carrier. In this respect, neuronal and mesenchymal stem cells have high migratory potential yet remain confined to tumor tissue. A subpopulation of adult mesenchymal cells (bone marrow derived tumor infiltrating cells or BM-TICs) has been shown, following injection into gliomas, to infiltrate the entire tumor. Thus, oncolytic viruses according to the invention can be administered in a virus-producing neuronal or mesenchymal stem cell (e.g. BM-TIC) carrier (e.g. by injection of the carrier cell into the tumor)

The quantity to be administered, both according to number of treatments and dose, depends on the subject to be treated, the state of the subject and the protection desired. Precise amounts of the therapeutic composition also depend on the judgment of the practitioner and are peculiar to each individual.

### Examples

The following examples as well as the figures are included to demonstrate preferred embodiments of the invention. It should be appreciated by those of skill in the art that the techniques disclosed in the examples or figures represent techniques discovered by the inventors to function well in the practice of the invention and thus can be considered to constitute preferred modes for its practice. However, those of skill in the art should, in light of the disclosure, appreciate that many changes can be made in the specific embodiments which are disclosed and still obtain a like or similar result without departing from the spirit and scope of the invention.

### Example 1- Construction and Characterization of Delta-24-RGDOX

The mouse OX40L expression cassette with CMV promoter replaced the E3 region of human adenovirus type 5 genome. A 24-bp sequence within the CR2 portion of the E1A gene (corresponding to amino acids 122-129 in the encoded E1A protein) responsible for binding Rb protein was deleted. A RGD-4C motif coding sequence is inserted in the HI-loop of fiber protein. See Figure 1.

Expression of mouse OX40L (mOX40L) by D24-RGDOX on GL261 (mouse glioma) and mouse melanoma B16 cells was assessed. GL261 or B16 cells were infected with D24-RGDOX at 50 pfu/cell. 48 hours later, the cells were stained with α-mOX40L antibody (1:100 dilution) (eBioscience, San Diego, CA) and then with FITC-labeled secondary antibody goat anti-rat IG (1:100 dilution) (Santa Cruz Biotechnology). The cell membrane integrity was monitored with ethidium homodimer -1 staining (8 µM) (Sigma-Aldrich, St. Louis, MO). The stained cells were analyzed with flow cytometry. The numbers at the lower right corners of Figures 2 and 3 indicate the percentage of GL261 and melanoma B16 cells expressing mOX40L. D24-RGDOX expressed OX40L efficiently on both GL261 cells and melanoma B16 cells.

Expression of mOX40L in GL261 -EGFP (Enhanced Green Fluorescent Protein-expressing GL261) tumor cells was assessed. GL261-EGFP cells (5 x 10⁴ cells) were injected intracranially in C57BL/6 mice. 12 days later, D24-RGDOX was injected intratumorally (5 x 10⁷ pfu). Three days after the injection the tumors were harvested and dissociated with ACCUMAX cell detachment solution (EMD Millipore, Billerica, MA). The cells were then stained with rat monoclonal α-mOX40L APC antibody (1:40) (eBioscience). The stained cells were analyzed with flow cytometry. Tumor cells were gated as EGFP positive. The numbers at the upper right corners of Figure 4 indicate the percentage of the tumor cells expressing mOX40L. These *in vivo* data demonstrate expression of OX40L in about 9% of the xenograft cells seventy-two hours after injection with D24-RGDOX.

Replication of D24-RGD and D24-RGDOX in U87 MG (human primary glioblastoma cell line with epithelial morphology; American Type Culture Collection, Manassas, VA) or GL261 cells was tested. Cells were seeded at a density of 5 x 10⁴ cells/well in 12-well plates and infected with the viruses at 10 pfu/cell. Forty-eight hours after infection, the infectious viral progeny were titered using the ADENO-X Rapid Titer Kit (Clontech, Mountain View, CA) according to manufacturer's instructions. Final viral titers were determined as pfu/ml and are shown in Figure 5 as mean ± SD of three independent measurements. The replication of the two viruses was compared using the Student's T-test (two-sided). D24-RGDOX was shown to replicate as efficiently as its parental virus D24-RGD in human glioma U-87 mg cells whereas both viruses replicate very poorly in GL261 cells. Thus, the antitumoral effects described herein with the mouse glioma model significantly under-represent the expected antitumoral effects of the virus (expressing OX40L) in humans.

The ability of D-24-RGD and D24-RGDOX to induce HSP90 and HMGB1 secretion was assessed. GL261 cells were infected with the viruses at 200 pfu/cell. 24 hours later, the concentration of the FBS was changed from 10% to 2%. Culture medium (M) and whole cell lysates (W) were collected at the time points indicated in Figure 6. Culture medium was concentrated 10-fold with Protein Concentrators (9K MWCO, Thermo Scientific). Then HSP90 and HMGB1 expression levels were analyzed with immunoblotting. Briefly, equal amounts of proteins from whole-cell lysates or 40 µl/lane concentrated medium were separated with 4-20% gradient sodium dodecyl sulfate-polyacrylamide gel electrophoresis, electrophoretically transferred to nitrocellulose membranes, and the membranes were probed with rabbit polyclonal anti-HSP90 and anti-HMGB1 (1:1000 dilution) (Cell Signaling Technology, Beverly, MA), goat polyclonal anti-actin (1:1000 dilution) (Santa Cruz Biotechnology, Santa Cruz, CA). The protein-antibody complexes were visualized using the enhanced chemiluminescence western blotting detection system (Amersham Pharmacia Biotech, Piscataway, NJ). Actin was used as a loading control for whole cell lysates. The numbers at the bottom of Figure 6 indicate the relative HMGB1 levels secreted to the medium. Despite the low replication efficiency of the virus in GL261 cells, both viruses induced the release of ATP and HMGB1, which are the prototype of endogenous damage-associated molecular pattern (DAMP) molecules that trigger inflammation and immunity during immunogenic cell death.

### Example 2 - Enhanced Therapeutic Effect Induced by D24-RGDOX

The effect of D24-RGDOX on survival of a glioma cancer model was assessed and compared to that of D24-RGD and OX86 (OX40 agonist) administered separately or together. GL261 cells (5 x 10⁴ cells) were injected intracranially in C57BL/6 mice and athymic mice. D24-RGDOX or D24-RGD (5 x 10⁷ pfu) and/or α-mouse OX40 antibody OX86 (25 µg, provided by the Monoclonal Antibody Core Facility at MDACC) were injected intratumorally on days 3, 6 and 8 after tumor implantation (the viruses were injected three times to partially compensate for the low replication of the viruses in GL261 cells). PBS was used as a negative control. Survival among treatment groups (PBS; D24-RGD; OX86; OX86+D24RGD; D24-RGDOX; n=10 in each group)_was compared using the log-rank test (two-sided). Figures 7A and 7B illustrate Kaplan-Meier curves of overall survival of the indicated treated groups (n = 10 each group) in C57BL/6 or athymic mice, respectively. This animal survival study demonstrated that, while D24-RGD itself showed no effect at the viral dose of 5 x 10⁷ pfu/mouse for each injection (p = 0.08), combination of D24-RGD with OX86 significantly prolonged the survival of the mice (median survival 24 days vs. 17 days, p = 0.0002). Importantly, D24-RGDOX further extended the median survival time to 28.5 days (p<0.0001) compared to D24-RGD. The prolonged survival of the mice is mainly due to the anti-glioma immunity triggered by the virus and the antibody because the therapeutic benefit was not observed in an immunodeficient GL261-athymic mouse glioma model (p>0.3) (Figure 7B).

The immune response induced by D24-RGDOX was examined and compared to that of D24-RGD using flow cytometry analysis. GL261 cells (5 x 10⁴ cells) were injected intracranially in C57BL/6 mice. The viruses (5 x 10⁷ pfu) were injected intratumorally on days 6, 8 and 10 after tumor implantation. On day 14, brain-infiltrated leukocytes (from group of 9 mice) were first separated from myelin debris with Percoll (GE Healthcare BioSciences, Pittsburgh, PA) gradient centrifuge and were directly used for flow cytometry analysis. The antibodies used were as follows: anti-mouse CD45 APC-EFLUOR 780 (1:200 dilution), anti-mouse CD3 FITC (1:200 dilution), anti-mouse CD8a PerCP-Cyanine5.5 (1:80 dilution) (eBioscience), BRILLIANT VIOLET 650 anti-mouse CD4 antibody (1:100 dilution) (BioLegend, San Diego, CA). Data are shown in Figure 8 as mean + SD of triplicate measurements. The cell numbers among treatment groups was compared using the Student's T-test (two-sided). The data demonstrate that D24-RGDOX was more efficient than D24-RGD to induce T lymphocytes (CD45+ CD3+), T helper cells (CD45+ CD3+ CD4+), cytotoxic T cells (CD45+ CD3+ CD8+) infiltration to the tumor sites (p < 0.001).

The effect of D24-RGDOX on anti-tumor immune response was assessed and compared to that of D24-RGD. GL261 cells (5 x 10⁴ cells) were injected intracranially in C57BL/6 mice. The viruses (5 x 10⁷ pfu) were injected intratumorally on days 6, 8, and 10 after tumor implantation. On day 14 after the tumor implantation, splenocytes from mouse spleens (group of 5 mice) of each treatment were isolated. For brain lymphocytes isolation (from group of 5 hemispheres with tumor), brain-infiltrated leukocytes were first separated from myelin debris as described above. Then, the brain lymphocytes were isolated with a gradient centrifuge in LYMPHOLYTE-M (Cedarlane, Burlington, NC). To activate the splenocytes, 2 x 10⁴ target cells pre-fixed with 1% paraformaldehyde (PFA) were incubated with 5 x 10⁵ brain infiltrated lymphocytes or splenocytes per well of a round-bottom 96-well plate for 40 hours. The concentration of IFNγ in the supernatant was assessed with standard ELISA assay (Mouse IFN-gamma DuoSet, R&D systems). Data are shown in Figure 9 as mean + SD of triplicate measurements. Figures 10A and 10B illustrate separate experiments in which brain infiltrated lymphocytes were isolated from the mice from each treatment group on day 21 after tumor implantation and co-cultured with MBCs as described above (Figure 10A) and in which splenocytes were isolated from the mice from each treatment group on day 21 after tumor implantation and co-cultured with the indicated target cells as described above (Figure 10B). In each case, the concentration of IFNγ in the supernatant was measured 40 hours later with standard ELISA assay (Mouse IFN-gamma DuoSet, R&D systems). Data are shown in Figures 10A and 10B as mean + SD of triplicate measurements. The activity among treatment groups was compared using the Student's T-test (two-sided). These data demonstrate that D24-RGDOX induced significantly stronger activity in the immune cells (spleenocytes and brain infiltrating lymphocytes (BILs)) against the uninfected or virus-infected tumor cells than D24-RGD or D24-RGD-EGFP (p ,0.05). Tumor cells infected with D24-RGDOX triggered stronger activity in BILs than the tumor cells infected with D24-RGD (p < 0.002) indicating that expression of OX40L by D24-RGDOX increased the capability of the tumor cells to stimulate the immune cells. Although D24-RGDOX caused stronger immune reaction against mouse brain cells (MBCs) primary culture than other groups in BILs (p = 0.01), it still induces significantly higher activity against tumor cells than against MBCs (p > 0.005). However, this increased reaction of BILs induced by D24-RGDOX against MBC (15.6 fold of D24-RGD) was acute since it was turned down after another seven days (1.6 fold of D24-RGD). The acute level of activity of BIL against MBCs induced by D24-RGDOX was reduced about four fold after seven days. In addition, the increased reaction against MBCs induced by D24-RGDOX was not observed in splenocytes (p = 0.2) while the increased reaction against tumor cells sustained after another seven days in splenocytes. The activity difference between D24-RGDOX-treated group and the other groups in splenocytes were even greater than seven days previous.

The present inventors, for the first time, have combined oncolytic adenovirus D24-RGD with targeting the late costimulatory OX40L/OX40 pathway to treat gliomas in an immunocompetent mouse model. D24-RGDOX displays superior capability to elicit anti-glioma immunity than its parental virus D24-RGD. Due to the cancer selective nature of D24-RGD, OX40L should be expressed preferentially on cancer cells. Moreover, unlike ligands for CD28 which also bind CTLA4, OX40 ligand selectively binds OX40. Thus, OX40L stimulates OX40 on T lymphocytes with TCR recognizing tumor-associated viral antigens, resulting in the expansion of tumor-specific T cell populations. Accordingly, different from OX40 agonist antibody, the antagonist antibodies for CTLA-4 and PD-1 or using oncolytic viruses to express immune modulators to globally activate immune cells, the modulation of T cells by OX40L expressed by D24-RGDOX is more limited to tumor-specific T cells. Therefore, D24-RGDOX is less likely to cause systemic toxicity related to those therapies. Based on the present exemplifications, it is expected that the percentage of human cancer patients with a complete response will be significantly increased with D24-RGDOX. The duration of the clinical response is also expected to increase with D24-RGDOX due to the enhanced immune memory stimulated by OX40L/OX40 pathway.

Embodiments of the invention will now be described in the following numbered paragraphs:
1. A replication competent oncolytic virus comprising a heterologous nucleic acid inserted into a nonessential region of the adenovirus genome, said nucleic acid comprising a sequence encoding an OX40 (CD134) agonist operatively linked to a transcriptional control element.
2. The replication competent oncolytic virus of paragraph 1, wherein the replication competent oncolytic virus is a replication competent oncolytic adenovirus.
3. The replication competent oncolytic adenovirus of paragraph 2, wherein the adenovirus comprises a deletion in part or all of the E3 gene region.
4. The replication competent oncolytic adenovirus of paragraph 3, wherein said heterologous nucleic acid is inserted in the E3 deleted gene region of the adenovirus.
5. The replication competent oncolytic adenovirus of any preceding paragraph, wherein the OX40 agonist is OX40 ligand (OX40L) (gp36).
6. The replication competent oncolytic adenovirus of paragraph 5, wherein the nucleic acid encoding OX40L encodes a polypeptide having the amino acid sequence set forth in GenBank Accession Number NP_003317.1 or a sequence at least 95% identical thereto.
7. The replication competent oncolytic adenovirus of paragraph 6, wherein the nucleic acid encoding OX40L has the nucleic acid sequence of NCBI Reference Sequence: NM_003326.3 or a sequence at least 95% identical thereto.
8. The replication competent oncolytic adenovirus of any preceding paragraph wherein the adenovirus is a human adenovirus type 5 or a hybrid comprising a human adenovirus type 5 component.
9. The replication competent oncolytic adenovirus of paragraph 8 wherein the adenovirus is Delta-24 or Delta-24-RGD.
10. The replication competent oncolytic adenovirus of any one of paragraphs 1-8 wherein the adenovirus is selected from ICOVIR-5, ICOVIR-7, ONYX-015, ColoAd1, H101 and AD5/3-D24-GMCSF.
11. The replication competent oncolytic adenovirus of any one of paragraphs 1-10 wherein the adenovirus genome comprises one or more heterologous nucleic acid sequences encoding a tumor antigen, whereby the adenovirus expresses the tumor antigen(s) on its surface.
12. The replication competent oncolytic adenovirus of paragraph 11 wherein the tumor antigen is selected from the group consisting of: MAGE-1, MAGE-2, MAGE-3, CEA, Tyrosinase, midkin, BAGE, CASP-8, β-catenin, CA-125, CDK-1, ESO-1, gp75, gplOO, MART-1, MUC-1, MUM-1, p53, PAP, PSA, PSMA, ras, trp-1, HER-2, TRP-1, TRP-2, IL13Ralpha, IL13Ralpha2, AIM-2, AIM-3, NY-ESO-1, C9orfl 12, SART1, SART2, SART3, BRAP, RTN4, GLEA2, TNKS2, KIAA0376, ING4, HSPH1, C13orf24, RBPSUH, C6orfl53, NKTR, NSEP1, U2AF1L, CYNL2, TPR, SOX2, GOLGA, BMI1, COX- 2, EGFRvIII, EZH2, LICAM, Livin, Livin, MRP-3, Nestin, OLIG2, ART1, ART4, B-cyclin, Glil, Cav-1, cathepsin B, CD74, E-cadherin, EphA2/Eck, Fra- 1/Fosl 1, GAGE-1, Ganglioside/GD2, GnT-V, β1,6-N, Ki67, Ku70/80, PROX1, PSCA, SOX10, SOX11, Survivin, UPAR and WT-1 or an immunogenic peptide thereof.
13. The replication competent oncolytic adenovirus of paragraph 12, wherein the heterologous nucleic acid is inserted in hyper-variable region 5 of the hexon gene of the adenovirus or is inserted into the HI loop region of the adenovirus fiber gene.
14. The replication competent oncolytic adenovirus of paragraph 12, wherein the adenovirus comprises a heterologous nucleic acid encoding EGFRvIII or an immunogenic peptide thereof inserted into the HI loop region of the fiber gene of the adenovirus and/or a heterologous nucleic acid encoding NY-ESO-1 or an immunogenic peptide thereof inserted in the hyper-variable region 5 of the hexon gene of the adenovirus.
15. A pharmaceutical composition comprising a replication competent oncolytic adenovirus according to any preceding paragraph and a pharmaceutically acceptable carrier.
16. The pharmaceutical composition of paragraph 15, further comprising one or more Th1 stimulating agents selected from the group consisting of: IL-12p70, IL-2, IFN-γ, lenalidomide, temozolomide (4-methyl-5-oxo- 2,3,4,6,8-pentazabicyclo [4.3.0] nona-2,7,9-triene- 9-carboxamide), cyclophosphamide ((RS)-N,N-bis(2-chloroethyl)-1,3,2-oxazaphosphinan-2-amine 2-oxide), lomustine (CCNU; N-(2-chloroethyl)-N'-cyclohexyl-N-nitrosourea), bis-chloroethylnitrosourea (BCNU), melphalan hydrochloride (4 [bis(chloroethyl)amino]phenylalanine), busulfan (butane-1,4-diyl dimethanesulfonate), mechlorethamine (nitrogen mustard), chlorambucil, ifosfamide, streptozocin, dacarbazine (DTIC), thiotepa, altretamine (hexamethylmelamine), cisplatin, carboplatin, oxalaplatin, Ipilimumab, Tremelimumab, MDX-1106, MK-3475, AMP-224, Pidilizumab, and MDX-1105.
17. The pharmaceutical composition of paragraph 16, wherein the Th1 stimulating agent is IFN-γ or temozolomide.
18. A method for treating cancer in a patient in need thereof, comprising administering to the patient a replication competent oncolytic adenovirus according to any of paragraphs 1-14 or a composition according to any one of paragraphs 15-17.
19. The method of paragraph 18, wherein the patient has a cancer selected from primary or metastatic brain cancer, melanoma, adenocarcinoma, thyoma, lymphoma, sarcoma, lung cancer, liver cancer, colon cancer, non-Hodgkins lymphoma, Hodgkins lymphoma, leukemia, uterine cancer, breast cancer, prostate cancer, ovarian cancer, cervical cancer, bladder cancer, kidney cancer, and pancreatic cancer.
20. The method of paragraph 19, wherein the patient has a low-level or high-level glioma.
21. The method of any of paragraphs 18-20, wherein the adenovirus is administered intratumorally, intravascularly, or in a neuronal or mesenchymal stem cell carrier.
22. The method of paragraph 21, wherein the adenovirus is administered intratumorally.
23. The method of any of paragraphs 18-22, wherein the adenovirus is administered once or multiple times at a dose of 10⁸-10¹³ plaque forming units (pfu).
24. The method of paragraph 22 or 23, comprising injection of an effective amount of the adenovirus into the tumor mass or vasculature.
25. The method of paragraph 24, whereby tumor growth is reduced in both the injected tumor and at least one non-injected tumor.
26. The method of any one of paragraphs 18-25, wherein the patient exhibits an IL-12 to IL-4 ratio less than 20.
27. A method for treating cancer in a patient in need thereof, comprising co-administering to the patient an effective combined amount of (i) a replication competent oncolytic adenovirus according to any of paragraphs 1-14 or a composition according to paragraph 15 and (ii) a Th1 stimulating agent.
28. The method of paragraph 27, wherein the Th1 stimulating agent is selected from the group consisting of: IL-12p70, IL-2, IFN-γ, lenalidomide, temozolomide (4-methyl-5-oxo-2,3,4,6,8-pentazabicyclo [4.3.0] nona-2,7,9-triene- 9-carboxamide), cyclophosphamide ((RS)-N,N-bis(2-chloroethyl)-l,3,2-oxazaphosphinan-2-amine 2-oxide), lomustine (CCNU; N-(2-chloroethyl)-N'-cyclohexyl-N-nitrosourea), bis-chloroethylnitrosourea (BCNU), melphalan hydrochloride (4 [bis(chloroethyl)amino]phenylalanine), busulfan (butane-1,4-diyl dimethanesulfonate), mechlorethamine (nitrogen mustard), chlorambucil, ifosfamide, streptozocin, dacarbazine (DTIC), thiotepa, altretamine (hexamethylmelamine), cisplatin, carboplatin, oxalaplatin, Ipilimumab, Tremelimumab, MDX-1106, MK-3475, AMP-224, Pidilizumab, and MDX-1105.
29. The method of paragraph 28, wherein the Th1 stimulating agent is IFN-γ or temozolomide.
30. The method of any one of paragraphs 27-29, wherein the Th1 stimulating agent is administered prior to the replication-competent oncolytic adenovirus.
31. The method of any one of paragraphs 27-30, wherein adenovirus is Delta-24 or Delta-24-RGD and the OX40 agonist is OX40 ligand (OX40L) (gp36).
32. The method of any one of paragraphs 18-31 wherein the patient is a human.

It should be understood that the invention is not limited to the embodiments set forth above, but is as defined in the appended claims.

## Claims

1. A replication competent oncolytic virus comprising a heterologous nucleic acid inserted into a nonessential region of the adenovirus genome, said nucleic acid comprising a sequence encoding an OX40 (CD134) agonist operatively linked to a transcriptional control element.

2. The replication competent oncolytic virus of claim 1, wherein the replication competent oncolytic virus is a replication competent oncolytic adenovirus.

3. The replication competent oncolytic adenovirus of claim 2, wherein the adenovirus comprises a deletion in part or all of the E3 gene region,
optionally wherein said heterologous nucleic acid is inserted in the E3 deleted gene region of the adenovirus.

4. The replication competent oncolytic adenovirus of any preceding claim, wherein the OX40 agonist is OX40 ligand (OX40L) (gp36),
optionally wherein the nucleic acid encoding OX40L encodes a polypeptide having the amino acid sequence set forth in GenBank Accession Number NP_003317.1 or a sequence at least 95% identical thereto and
also optionally wherein the nucleic acid encoding OX40L has the nucleic acid sequence of NCBI Reference Sequence: NM_003326.3 or a sequence at least 95% identical thereto.

5. The replication competent oncolytic adenovirus of any preceding claim wherein the adenovirus is a human adenovirus type 5 or a hybrid comprising a human adenovirus type 5 component,
optionally wherein the adenovirus is Delta-24 or Delta-24-RGD.

6. The replication competent oncolytic adenovirus of any one of claims 1-5 wherein the adenovirus is selected from ICOVIR-5, ICOVIR-7, ONYX-O15, ColoAd1, H101 and AD5/3-D24-GMCSF.

7. The replication competent oncolytic adenovirus of any one of claims 1-6 wherein the adenovirus genome comprises one or more heterologous nucleic acid sequences encoding a tumor antigen, whereby the adenovirus expresses the tumor antigen(s) on its surface,
optionally wherein the tumor antigen is selected from the group consisting of: MAGE-1, MAGE-2, MAGE-3, CEA, Tyrosinase, midkin, BAGE, CASP-8, β-catenin, CA-125, CDK-1, ESO-1, gp75, gplOO, MART-1, MUC-1, MUM-1, p53, PAP, PSA, PSMA, ras, trp-1, HER-2, TRP-1, TRP-2, IL13Ralpha, IL13Ralpha2, AIM-2, AIM-3, NY-ESO-1, C9orfl 12, SART1, SART2, SART3, BRAP, RTN4, GLEA2, TNKS2, KIAA0376, ING4, HSPH1, C13orf24, RBPSUH, C6orfl53, NKTR, NSEP1, U2AF1L, CYNL2, TPR, SOX2, GOLGA, BMI1, COX-2, EGFRvIII, EZH2, LICAM, Livin, Livin , MRP-3, Nestin, OLIG2, ART1, ART4, B-cyclin, Glil, Cav-1, cathepsin B, CD74, E-cadherin, EphA2/Eck, Fra- 1/Fosl 1, GAGE-1, Ganglioside/GD2, GnT-V, β1,6-N, Ki67, Ku70/80, PROX1, PSCA, SOX10, SOX11, Survivin, UPAR and WT-1 or an immunogenic peptide thereof.

8. The replication competent oncolytic adenovirus of claim 7, wherein the heterologous nucleic acid is inserted in hyper-variable region 5 of the hexon gene of the adenovirus or is inserted into the HI loop region of the adenovirus fiber gene,
optionally wherein the adenovirus comprises a heterologous nucleic acid encoding EGFRvIII or an immunogenic peptide thereof inserted into the HI loop region of the fiber gene of the adenovirus and/or a heterologous nucleic acid encoding NY-ESO-1 or an immunogenic peptide thereof inserted in the hyper-variable region 5 of the hexon gene of the adenovirus.

9. A pharmaceutical composition comprising a replication competent oncolytic adenovirus according to any preceding claim and a pharmaceutically acceptable carrier, optionally
further comprising one or more Th1 stimulating agents selected from the group consisting of: IL-12p70, IL-2, IFN-γ, lenalidomide, temozolomide (4-methyl-5-oxo- 2,3,4,6,8-pentazabicyclo [4.3.0] nona-2,7,9-triene- 9-carboxamide), cyclophosphamide ((RS)-N,N-bis(2-chloroethyl)-1,3,2-oxazaphosphinan-2-amine 2-oxide), lomustine (CCNU; N-(2-chloroethyl)-N'-cyclohexyl-N-nitrosourea), bis-chloroethylnitrosourea (BCNU), melphalan hydrochloride (4 [bis(chloroethyl)amino]phenylalanine), busulfan (butane-1,4-diyl dimethanesulfonate), mechlorethamine (nitrogen mustard), chlorambucil, ifosfamide, streptozocin, dacarbazine (DTIC), thiotepa, altretamine (hexamethylmelamine), cisplatin, carboplatin, oxalaplatin, Ipilimumab, Tremelimumab, MDX-1106, MK-3475, AMP-224, Pidilizumab, and MDX-1105.

10. The pharmaceutical composition of claim 9 wherein the Th1 stimulating agent is IFN-γ or temozolomide.

11. A replication competent oncolytic adenovirus according to any of claims 1-8 or a pharmaceutical composition according to claims 9 and 10 for use in treating cancer in a patient in need thereof.

12. The replication competent oncolytic adenovirus or composition for use of claim 11, wherein:
a) the patient has a cancer selected from primary or metastatic brain cancer, melanoma, adenocarcinoma, thyoma, lymphoma, sarcoma, lung cancer, liver cancer, colon cancer, non-Hodgkins lymphoma, Hodgkins lymphoma, leukemia, uterine cancer, breast cancer, prostate cancer, ovarian cancer, cervical cancer, bladder cancer, kidney cancer, and pancreatic cancer,
optionally wherein the patient has a low-level or high-level glioma,
b) the adenovirus is administered intratumorally, intravascularly, or in a neuronal or mesenchymal stem cell carrier,
optionally wherein the adenovirus is administered intratumorally,
c) the adenovirus is administered once or multiple times at a dose of 10⁸-10¹³ plaque forming units (pfu),
d) comprising injection of an effective amount of the adenovirus into the tumor mass or vasculature,
optionally whereby tumor growth is reduced in both the injected tumor and at least one non-injected tumor,
e) the patient exhibits an IL-12 to IL-4 ratio less than 20.

13. A replication competent oncolytic adenovirus according to any of claims 1-8 or a pharmaceutical composition according to claim 9 combined with a Th1 stimulating agent for use in treating cancer in a patient in need thereof,
optionally wherein the Th1 stimulating agent is selected from the group consisting of: IL-12p70, IL-2, IFN-γ, lenalidomide, temozolomide (4-methyl-5-oxo- 2,3,4,6,8-pentazabicyclo [4.3.0] nona-2,7,9-triene- 9-carboxamide), cyclophosphamide ((RS)-N,N-bis(2-chloroethyl)-1,3,2-oxazaphosphinan-2-amine 2-oxide), lomustine (CCNU; N-(2-chloroethyl)-N'-cyclohexyl-N-nitrosourea), bis-chloroethylnitrosourea (BCNU), melphalan hydrochloride (4 [bis(chloroethyl)amino]phenylalanine), busulfan (butane-1,4-diyl dimethanesulfonate), mechlorethamine (nitrogen mustard), chlorambucil, ifosfamide, streptozocin, dacarbazine (DTIC), thiotepa, altretamine (hexamethylmelamine), cisplatin, carboplatin, oxalaplatin, Ipilimumab, Tremelimumab, MDX-1106, MK-3475, AMP-224, Pidilizumab, and MDX-1105;
more optionally wherein the Th1 stimulating agent is IFN-γ or temozolomide.

14. The replication competent oncolytic adenovirus or pharmaceutical composition combined with a Th1 stimulating agent for use of claim 13, wherein the Th1 stimulating agent is administered prior to the replication-competent oncolytic adenovirus, and/or wherein adenovirus is Delta-24 or Delta-24-RGD and the OX40 agonist is OX40 ligand (OX40L) (gp36).

15. The replication competent oncolytic adenovirus or pharmaceutical composition for use of claim 11 or 12 or the replication competent oncolytic adenovirus or pharmaceutical composition combined with a Th1 stimulating agent for use of claim 13 or 14, wherein the patient is a human.
